(19) Europäisches Patentamt
European Patent Office
Office européen des brevets

(11) Veröffentlichungsnummer: 0 335 381
A1

# (12) EUROPÄISCHE PATENTANMELDUNG

(21) Anmeldenummer: 89105570.9

(22) Anmeldetag: 29.03.89

(51) Int. Cl.4: C07D 233/54 , C07D 249/08 , A61K 31/41 , C07D 409/14 , C07D 401/12 , C07D 403/12 , C07D 401/14

(30) Priorität: 30.03.88 DE 3810848

(43) Veröffentlichungstag der Anmeldung: 04.10.89 Patentblatt 89/40

(84) Benannte Vertragsstaaten: AT BE CH DE ES FR GB GR IT LI LU NL SE

(71) Anmelder: BOEHRINGER INGELHEIM KG
Postfach 200
D-6507 Ingelheim am Rhein(DE)

(84) BE CH DE ES FR GR IT LI LU NL SE AT

Anmelder: BOEHRINGER INGELHEIM INTERNATIONAL G.M.B.H.
Postfach 20
D-6507 Ingelheim am Rhein(DE)

(84) GB

(72) Erfinder: Schromm, Kurt, Dr.
In der Dörrwiese 35
D-6507 Ingelheim am Rhein(DE)
Erfinder: Mentrup, Anton, Dr.
Biebricher Allee 15
D-6200 Wiesbaden(DE)
Erfinder: Renth, Ernst-Otto, Dr.
Frankenstrasse 11
D-6507 Ingelheim am Rhein(DE)
Erfinder: Heuer, Hubert, Dr.
Am Alten Weg 29
D-6500 Mainz 32(DE)
Erfinder: Muacevic, Gojko, Dr.
In der Dörrwiese 13
D-6507 Ingelheim am Rhein(DE)
Erfinder: Birke, Franz, Dr.
Albrecht-Dürer-Strasse 23
D-6507 Ingelheim am Rhein(DE)

(54) Neue 2,3,4-substituierte Imidazole und 3,4,5-substituierte 1,2,4-Triazole, ihre Herstellung und Verwendung.

(57) Die neuen Verbindungen der Formel

EP 0 335 381 A1

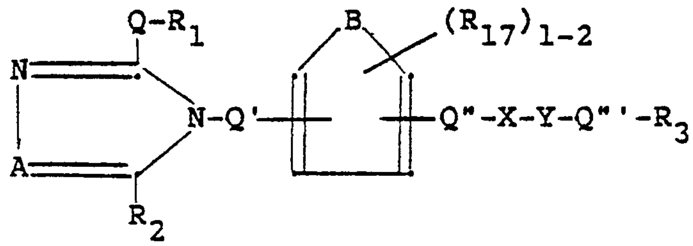

(Ia)

bzw.

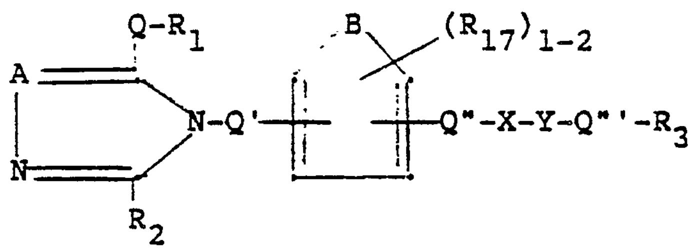

(Ib)

(die Symbole A, B, Q - Q''', $R_1$, $R_2$, $R_3$, X und Y sind in der Beschreibung definiert) können nach üblichen Methoden hergestellt und als Arzneistoffe verwendet werden.

## Neue 2,3,4-substituierte Imidazole und 3,4,5-substituierte 1,2,4-Triazole, ihre Herstellung und Verwendung

Die Erfindung betrifft neue 2,3,4-substituierte Imidazole und 3,4,5-trisubstituierte 1,2,4-Triazole, ihre Herstellung mit an sich bekannten Methoden und ihre Verwendung als Wirkstoffe in Arzneimitteln.

Die neuen Verbindungen entsprechen der Formel

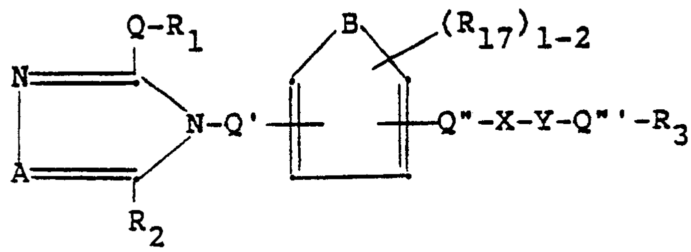

(Ia)

bzw.

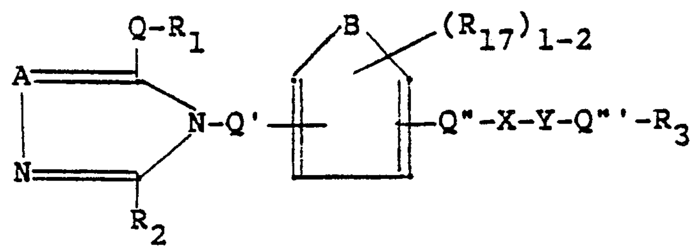

(Ib)

Darin steht

A für N oder CH;

B für einen ein- oder zweigliedrigen Ringbestandteil eines ein- oder mehrkernigen aromatischen oder heteroaromatischen Ringsystems, insbesondere eines solchen, in dem B CH=CH, S, O, $NR_{16}$ bedeutet;

Q, Q′, Q″, Q‴ für eine Einfachbindung oder $C_1$-$C_3$-Alkylen, Q zusätzlich O oder $NR_{16}$;

$R_1$ für

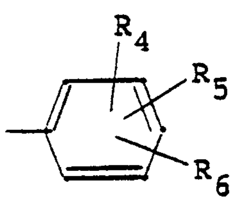

(II),

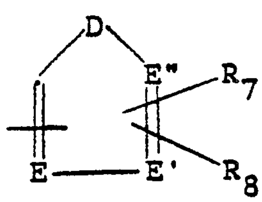

(III),

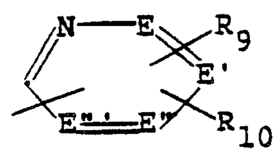

(IV);

wobei

$R_4$, $R_5$: H, Halogen, $R_{11}$, $OR_{11}$, Aryl, O-Aryl, Aralkyl, O-Aralkyl, $N(R_{11})_2$,

$R_4$ und $R_5$ gemeinsam auch einen gegebenenfalls substituierten ankondensierten $C_5$-$C_7$-Cycloalkenring oder ankondensierten Benzolring oder eine der an benachbarte C-Atome des Benzolrings gebundenen Gruppen -N=CH-NH-, -N=CH-CH=CH-, $-O-CH_2-CH_2-$, $-O-CH_2-CH_2-CH_2-$, $-O-(CH_2)_{1-3}-O-$, -NH-CO-NH-, -N=CH-CH=N-, $-HN-CO-C(R_{16})_2-O-$, -HN-CO-O-, $-NH-CO-CH_2$, -HN-CO-CH=CH--HN-CO-$CH_2$-$CH_2$-;

$R_6$: H, Halogen, $R_{11}$, $OR_{11}$ oder Aryl;

$R_7$: H, Halogen, $R_{11}$, $OR_{11}$, Aryl oder Aralkyl;

$R_8$: H, Halogen, $R_{11}$, $OR_{11}$ Aryl, Aralkyl, $N(R_{11})_2$,

$R_7$ und $R_8$ gemeinsam auch einen ankondensierten gegebenenfalls substituierten $C_5$-$C_7$-Cycloalken-

oder Benzolring,

$R_9$,$R_{10}$: H, Halogen, $R_{11}$, $OR_{11}$, $N(R_{11})_2$, Aryl, O-Aryl, Aralkyl, O-Aralkyl,

$R_9$ und $R_{10}$ gemeinsam auch einen gegebenenfalls substituierten ankondensierten $C_5$-$C_7$-Cycloalken- oder Benzolring;

D: S, O, $NR_{16}$;

E,E′,E″,E‴ CH, N, wobei im Fall der Gruppe III nicht mehr als zwei, im Fall der Gruppe IV nicht mehr als drei der Symbole E - E‴ für N stehen;

$R_{11}$: H, $C_1$-$C_{12}$-Alkyl, das sauerstoffunterbrochen oder substituiert sein kann durch bis zu 5 Halogenatome, $N(R_{16})_2$, Aryl, O-Aryl, einen $C_3$-$C_7$-Cycloaliphaten oder einen N-Heterocyclus, der über das Ringstickstoffatom an das Alkyl gebunden ist und der als weiteres Heteroringglied O, S, oder $NR_{16}$- enthalten kann; Alkenyl oder Alkinyl mit insgesamt bis zu 6 C-Atomen;

$R_2$ für H, OH, O-Acyl (mit bis zu 7 C-Atomen), einen gesättigten oder ungesättigten aliphatischen Rest mit bis zu 8 C-Atomen, der O-, S- oder $NR_{16}$ unterbrochen und durch bis zu fünf Halogenatome, OH, O-Acyl, Oxo, Aryl oder O-Aryl substituiert sein kann und der auch über Sauerstoff an den Imidazol- bzw. Triazolring gebunden sein kann; für einen gegebenenfalls durch $R_{16}$, OH, Oxo, $N(R_{16})_2$, substituierten, gesättigten oder ungesättigten, gegebenenfalls über Sauerstoff oder $C_1$-$C_4$-Alkylen an den Imidazol- bzw. Triazolring gebundenen $C_3$-$C_7$-cycloaliphatischen Ring; oder für eine Gruppe $(CH_2)_n$-$NR_aR_b$ (n = 0, 1, 2 oder 3), $R_a$ und $R_b$ H, oder einen gesättigten oder ungesättigten, gegebenenfalls sauerstoffunterbrochenen aliphatischen Rest mit bis zu 6 C-Atomen, der auch OH oder $N(R_{16})_2$ substituiert sein kann; $R_a$ außerdem für einen $C_3$-$C_7$-cycloaliphatischen Rest; die ganze Gruppe $NR_aR_b$ auch für einen 5-7-gliedrigen Ring der als zusätzliches Ringglied O, S oder $NR_{16}$ enthalten kann;

$R_3$ für einen ein- oder mehrkernigen gegebenenfalls substituierten vorzugsweise aromatischen carbocyclischen oder stickstoffhaltigen heterocyclischen Rest steht, wobei letzterer über ein Stickstoff- oder ein Kohlenstoffatom an -Q‴-Y- gebunden sein kann, insbesondere für

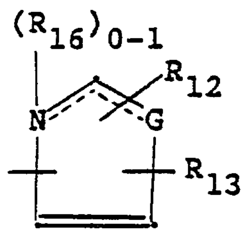

(V)

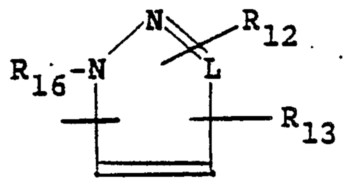

(V')

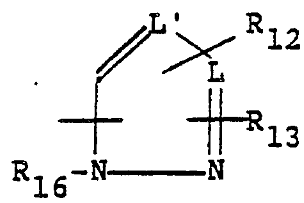

(V")

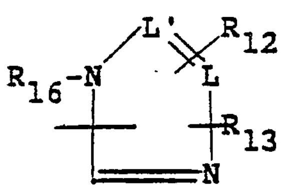

(V"')

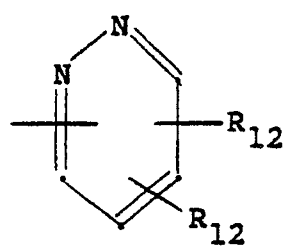

(VI)

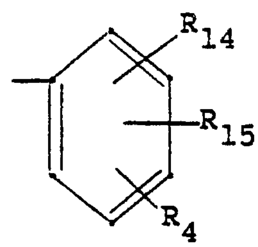

(VII)

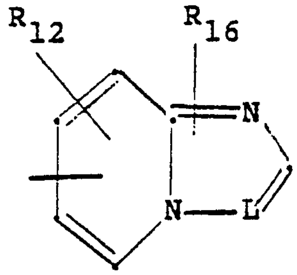 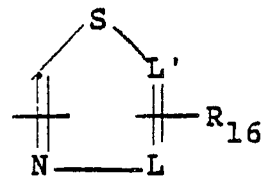 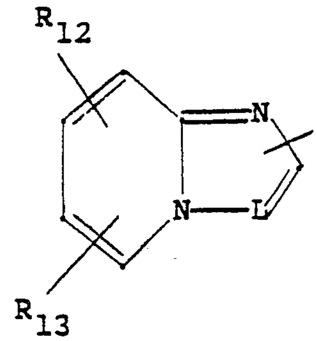

(VIII) (IX) (X)

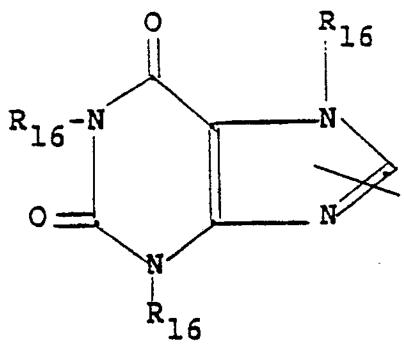 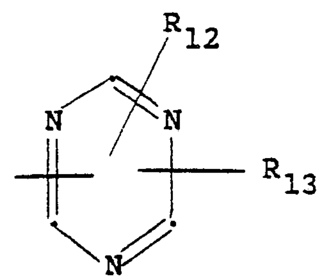

(X') (X")

wobei

G: S, O, CH, CH = CH, N = CH, CH = N, N = N, $NR_{16}$;

L,L': N, $CR_{16}$;

$R_{12}$: H, $(O)_{0-1}$-($C_1$-$C_4$-Alkyl), Aryl, O-Aryl, Aralkyl, Halogen, OH,

$R_{13}$: H, $(O)_{0-1}$-($C_1$-$C_4$-Alkyl), wobei die Alkylketten jeweils OH- oder bis zu fünffach halogensubstituiert sein können, Alkenyl- oder Alkinyl mit bis zu 6 C-Atomen oder Halogen, $R_{12}$ und $R_{13}$ gemeinsam auch einen gegebenenfalls substituierten ankondensierten Benzolring,

$R_{14}$: H, Halogen, CN, OH, $(O)_{0-1}$-($C_1$-$C_4$-Alkyl) (wobei das Alkyl durch bis zu 5 Halogenatome substituiert sein kann), $N(R_{16})_2$, Alkenyl- oder Alkinylreste mit bis zu 6 C-Atomen, CO-($C_1$-$C_4$-Alkyl), CO-Aryl, Aralkyl, Aryl, O-Aryl, ein Rest der Formel III oder IV;

$R_{15}$: H, Halogen, OH, $(O)_{0-1}$-($C_1$-$C_4$-Alkyl), gegebenenfalls durch OH oder bis zu 5 Halogenatome substituiert, $R_{14}$ und $R_{15}$ gemeinsam auch einen gegebenenfalls substituierten ankondensierten homocyclischen oder heterocyclischen Fünf- bis Siebenring, wobei bis zu zwei Ringglieder Heteroatome aus der Gruppe N, $NR_{16}$, O und S sein können;

$R_{16}$: H, $C_1$-$C_4$-Alkyl;

$R_{17}$ für H, $(O)_{0-1}$-$C_1$-$C_4$-Alkyl, Halogen;

X-Y für eine Einfachbindung, eine zweibindige Brückengruppe der Formel $CONR_{16}$ $CSNR_{16}$,C(NH)-$NR_{16}$,$NR_{16}CO$, $SO_2NR_{16}$ $NR_{16}SO_2$, $CONR_{16}NR_{16}$, $NR_{16}CONR_{16}$, $NR_{16}C(NR_{16})NR_{16}$, $NR_{16}CONR_{16}NR_{16}$, $CO(CH_2)_{1-3}NH$, $NH(CH_2)_{1-3}CO$, $SO_2(CH_2)_{1-3}NH$, $NH(CH_2)_{1-3}SO_2$, und, wenn der Rest $R_3$ über ein Kohlenstoffatom mit Q‴ verknüpft ist und mindestens eine der Gruppen Q″ und Q‴ für eine Einfachbindung steht, auch CO oder O.

Die Verbindungen können gegebenenfalls in Form einzelner räumlicher Isomerer und ihrer Mischungen und/oder von Säureadditionssalzen vorliegen.

Bevorzugt haben die Symbole im Rahmen der obigen Definitionen folgende Bedeutung:

B: CH = CH, S, $NR_{16}$ oder O;

$R_1$: eine Gruppe der Formel II, worin

$R_4$, $R_5$, $R_6$ . H, $C_1$-$C_4$-Alkyl, $C_1$-$C_4$-Alkoxy oder Halogen bedeuten, $R_4$ außerdem Aryl, Aralkyl, Aryloxy oder $N(R_{16})_2$ sowie $C_5$-$C_{12}$-Alkyl oder -Alkoxy, wenn $R_5$ und $R_6$ H sind, ferner $R_4$ und $R_5$ gemeinsam ankondensiertes $C_5$-$C_7$-Cycloalken, O-$(CH_2)_{1-3}$-O oder N=CH-NH (gebunden an benachbarte C-Atome), und, wenn $R_6$ Wasserstoff ist, auch einen ankondensierten Benzolring;

eine Gruppe der Formel III, worin

D die obige Bedeutung hat, höchstens eines der Symbole E, E′, E″, E‴ N bedeutet, während die übrigen CH sind;

$R_7$, $R_8$ H, $C_1$-$C_4$-Alkyl, $C_1$-$C_4$-Alkoxy, Halogen, ferner, für $R_8$ gleich H, $R_7$ auch $C_5$-$C_{12}$-Alkyl oder Aryl, $R_7$ und $R_8$ gemeinsam auch einen ankondensierten $C_5$-$C_7$-Cycloalken- oder Benzolring bedeuten;

eine Gruppe der Formel IV, worin von den Symbolen E, E′, E″, E‴ nicht mehr als zwei N bedeuten;

$R_9$, $R_{10}$ Halogen, $R_{16}$, $OR_{16}$, $N(R_{16})_2$,

$R_9$ auch Aryl, O-Aryl, Aralkyl und $R_9$ und $R_{10}$ gemeinsam auch einen ankondensierten $C_5$-$C_7$-Cycloalkenring oder Benzolring bedeuten;

$R_2$: H, OH, gesättigter oder ungesättigter aliphatischer Rest mit bis zu 8 C-Atomen, $C_3$-$C_6$-Cycloalkyl, bis zu fünffach halogensubstituiertes $C_1$-$C_3$-Alkyl oder $CH_3OCH_2CH_2$,

$R_3$: eine Gruppe der Formel V, V′, V″, V‴, VII oder IX,

worin

G, L, L′, $R_{16}$ und $R_{17}$ die obige Bedeutung haben;

$R_{11}$: $R_{16}$ ist;

$R_{12}$: $R_{16}$, Aryl oder Halogen,

$R_{13}$: $R_{16}$, Alkenyl mit bis zu 4 C-Atomen,

$R_{12}$ und $R_{13}$ gemeinsam auch einen ankondensierten Benzolring bedeuten;

$R_{14}$: $R_{16}$, Halogen, CN, $CF_3$, CO-($C_1$-$C_4$-Alkyl), $OR_{16}$;

$R_{15}$: $R_{16}$, Halogen oder $OR_{16}$;

$R_4$: die oben als bevorzugt genannte Bedeutung hat;

A, Q, Q′, Q″, Q‴sowie XY sind wie oben definiert.

Soweit die erfindungsgemäßen Verbindungen einen Pyridinring umfassen, insbesondere in $R_3$, kann dessen Stickstoffatom in quaternisierter Form vorliegen. Als zusätzliche Gruppe trägt das N-Atom einen Niederalkyl- oder Aralkylrest. Hervorzuheben sind $C_1$-$C_4$-Alkyl und Benzyl, so daß beispielsweise Verbindungen wie

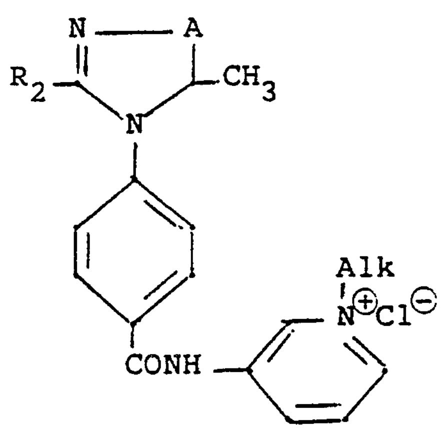

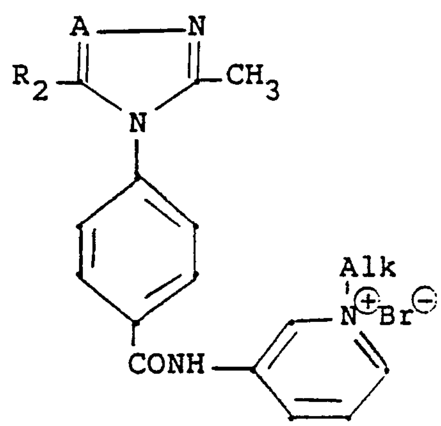

vorliegen können, worin A die obige Bedeutung hat, Alk z.B. $CH_3$, n-$C_3H_7$ und $R_2$ die zuvor angegebene Bedeutung hat, z.B. 5-Brom- oder 5-Methylthienyl oder 3-Chlor-, 3,5-Dichlor- oder 3,5-Dimethoxyphenyl ist.

In Formel V steht eine (und nur eine) der gestrichelten Linien für eine Doppelbindung. Geht sie von dem Stickstoffatom aus, fällt $(R_{16})_{0-1}$ weg, d.h. der Index hat den Wert "O".

Besonders hervorzuheben sind im Rahmen der obigen Definitionen die Verbindungen, in denen

A und D die obige Bedeutung haben;

B für CH=CH oder S steht;

Q und Q′ eine Einfachbindung oder $CH_2$ darstellen,

Q″ $C_1$-$C_3$-Alkylen, vorzugsweise jedoch eine Einfachbindung ist;

Q‴ eine Einfachbindung oder, falls XY CO ist, auch $CH_2$;

$R_1$ für die Gruppen II und III steht, wobei

$R_4$, $R_5$, und $R_6$ die oben als bevorzugt genannte Bedeutung haben, ebenso D, E, E′, E″, G, L, L″, $R_3$, $R_{11}$,

$R_{14}$, $R_{15}$, $R_{16}$;
$R_7$, $R_8$ H, $C_1$-$C_4$-Alkyl, $C_1$-$C_4$-Alkoxy, $R_8$ auch Aryl, $R_7$ und $R_8$ gemeinsam auch einen ankondensierten Benzolring oder $C_5$-$C_7$-Cycloalkenring darstellen;
$R_2$ für H, $C_1$-$C_3$-Alkyl, Cyclopropyl, Phenyl, $CF_3$ oder Allyl steht;
$R_3$ für eine der Gruppen V, V′, V″, V‴, VII oder IX steht, wobei V bevorzugt

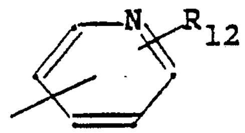

ist;
$R_{12}$ für $R_{16}$, Phenyl, Halogen;
$R_{13}$ für $R_{16}$ oder gemeinsam mit $R_{12}$ auch für einen ankondensierten Benzolring steht;
$R_{17}$ H, $CH_3$, $OCH_3$ oder Cl ist,
XYQ‴ für $CONR_{16}$, $NR_{16}CO$, $SO_2NR_{16}$, $NR_{16}SO_2$, $NR_{16}CONR_{16}$, $CO(CH_2)_{1-3}NH$, $COCH_2$ oder O steht.

Die neuen Verbindungen können als freie Basen oder als Säureadditionssalze vorliegen.

Weiterhin hervorzuheben sind die Verbindungen der Formel

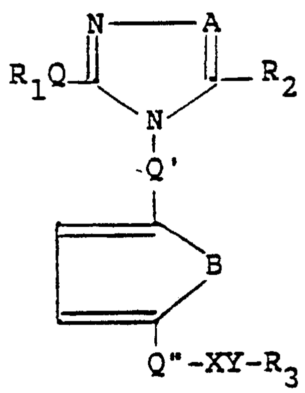

(I'a)

und

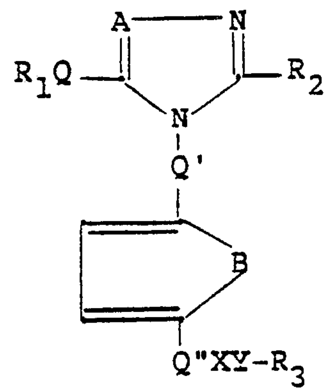

(I'b)

A: CH,N;
B: CH=CH, S;
Q, Q′, Q″: Einfachbindung, $CH_2$;
$R_1$:

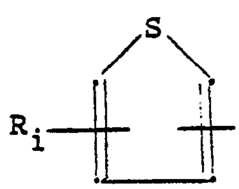

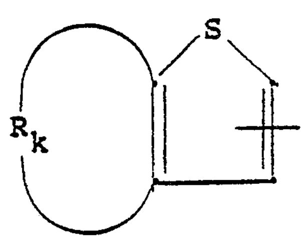

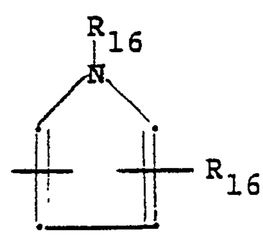

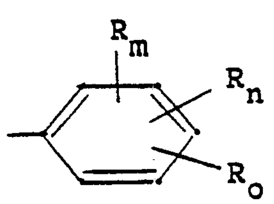

$R_i$: $C_1$-$C_4$-Alkyl, Cl, Br, $C_3$-$C_7$-Cycloalkyl;
$R_k$: ankondensierter $C_5$-$C_6$-Cycloalken- oder Benzolring;

$R_m$, $R_n$, $R_o$: H, Cl, $CH_3$, $C_1$-$C_4$-Alkoxy, $CF_3$, $N(R_{16})_2$, für den Fall $R_o$ gleich H sind $R_m$ und $R_n$ gemeinsam außerdem ein ankondensierter Benzolring oder $OCH_2O$, gebunden an benachbarte C-Atome;

$R_{16}$: H, $C_1$-$C_4$-Alkyl, $N(R_{16})_2$ vor allem $N(CH_3)_2$ und $N(C_2H_5)_2$;

$R_2$: $CH_3$, $C_2H_5$;

XY: CONH, NHCO, $SO_2NH$, NHCONH, $COCH_2$, $CH_2O$;

$R_3$: 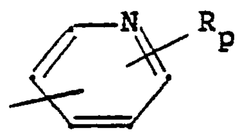 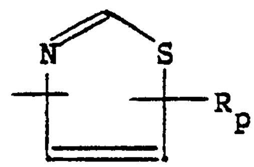 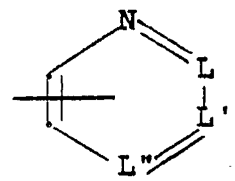

wobei eines der Symbole L, L′, L″ für N, die anderen für CH stehen und $R_p$ H oder $CH_3$ ist.

Weiter hervorzuheben sind die nachstehenden Verbindungen:

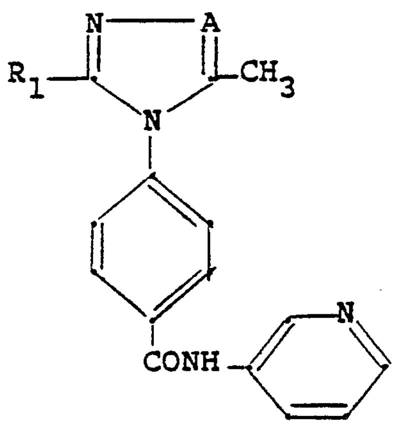

(I"a)

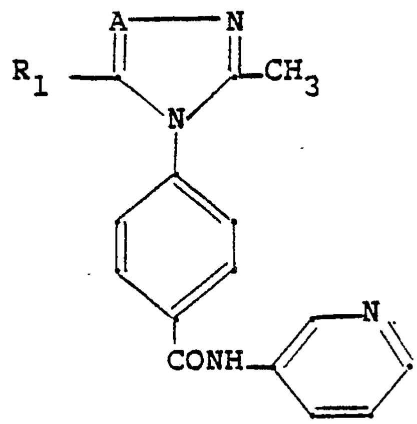

(I"b)

worin

$R_1$: Naphthyl sowie

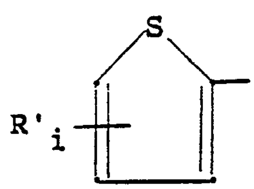 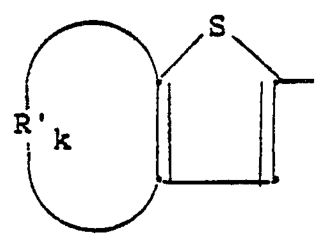 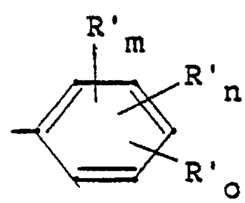

A: N oder CH

$R'_i$: $C_1$-$C_4$-Alkyl, Cl, Br,

$R'_k$: ankondensierter Cyclopenten- oder Cyclohexenring;

$R'_m$: H, Cl, $CH_3O$, Cl, $CF_3$, $CH_3$;

$R'_n$: H, $CH_3O$, Cl, $CH_3$; beide zusammen $OCH_2O$ an benachbarten C-Atomen;

$R'_o$: H, $CH_3O$, $CH_3$.

Für die obigen Definitionen gilt folgendes:

Soweit Symbole in einer Formel mehrfach vorkommen, können sie gleiche oder verschiedene Bedeutungen haben.

Die Kohlenwasserstoffketten können - wenn nichts anderes angegeben ist - unverzweigt oder verzweigt

sein. "Halogen" bedeutet Fluor, Chlor, Brom und auch Jod; als Halogenatome in aliphatischen Resten sind Fluor und Chlor hervorzuheben. Entsprechende Reste sind z.B. $CF_3$, $OCF_3$, $CF_2CF_3$, $CClF_2$. "Aryl" ist bevorzugt Phenyl oder Naphthyl. Als "Heteroaryl" sind auch solche Reste zu betrachten, die aus aromatischen und heteroaromatischen Ringen zusammengesetzt sind, z.B. Chinolin, Chinazolin. Als "Aralkyl" werden solche Aryle bzw. Heteroaryle bezeichnet, die über eine geradkettige oder verzweigte Alkylenbrükke gebunden sind.

Die aromatischen bzw. heteroaromatischen Gruppen können ein- oder mehrfach substituiert sein und können jeweils gleiche oder verschiedene Substituenten aufweisen. Substituenten sind Halogen, $C_1$-$C_4$-Alkyl, $C_1$-$C_4$-Alkoxy (die auch halogensubstituiert sein können), $N(R_{16})_2$, $NO_2$, niedere Alkenyl- oder Alkinylreste, CN, Aryl, $C_3$-$C_7$-Cycloalkyl, $COOR_{16}$, $CON(R_{16})_2$, $SO_2N(R_{16})_2$, O-Aryl, O-Aralkyl, $NHSO_2$- ($C_1$-$C_4$-Alkyl), OH, $NR_{16}COR_{18}$ (wobei $R_{18}$ einen aliphatischen oder cycloaliphatischen, Rest mit bis zu 7 C-Atomen, $N(R_{16})_2$ oder H bedeutet. Als "nieder" sind Reste mit bis zu 6, vorzugsweise bis zu 4 C-Atomen bezeichnet.

Während Halogen, Alkyl oder Alkoxy bis zu dreimal an einem Ring als Substituenten vorkommen können (im Ausnahmefall sind am Phenyl auch vier oder fünf Substituenten möglich), kommen die übrigen Substituenten im allgemeinen nur ein- bis zweimal vor. Die Gesamtzahl der Substituenten beträgt im allgemeinen nicht mehr als drei an einem Ring.

Im Fall von Halogenatomen, gegebenenfalls in Mischung mit Alkyl- oder Alkoxyresten, können gegebenenfalls auch bis zu 5 Substituenten an einem Ring vorhanden sein. Es versteht sich, daß die Substitutionsmöglichkeiten durch Heteroatome im Ring verrringert werden können. Wenn beispielsweise die Gruppe IV den Rest

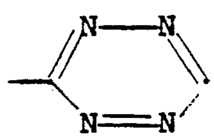

bedeutet, kann nur ein Substituent (d.h. $R_9$) vorhanden sein

Als Substituenten im Sinne der vorstehenden Ausführungen sind speziell zu nennen: F, Cl, Br, $CH_3$, $C_2H_5$, t-$C_4H_9$, n-$C_3$-$H_7$, i-$C_3H_7$, $OCH_3$, $OC_2H_5$, $CF_3$, $OCF_3$, Benzyl, Cyclopropyl, Phenyl, Allyl, Propargyl, $COOCH_3$, $COOC_2H_5$, $CON(C_2H_5)_2$, $SO_2N(CH_3)_2$, $NHSO_2CH_3$, $NHCOCH_3$, $NHCONH_2$, Phenoxy.

Alkenyl- und Alkinylreste sind in der Regel über ein gesättigtes C-Atom gebunden.

Soweit in den für X-Y angegebenen Gruppen $R_{16}$ enthalten ist, ist dessen Bedeutung vorzugsweise H.

Soweit $R_{11}$ als Substituent in den Gruppen, II, III und IV neben anderen Substituenten vorkommt, beschränkt sich die Kettenlänge auf vorzugsweise bis zu 4 Glieder. Soweit $R_{11}$ einen Heterocyclus umfaßt, ist dieser ein fünf- bis siebengliedriger, vorzugsweise nichtaromatischer Ring, z.B. Pyrrolidin, Piperidin, Piperazin, Morpholin, wobei diese Ringe z.B. niederalkylsubstituiert sein können.

Als typische Vertreter der Gruppen II bis IV seien erwähnt:

Phenyl, Benzyl, 3-Methoxy-, Dimethylamino- oder 3-Chlorphenyl, 3,5-Dimethoxy-, 3,5-Dimethyl-, 3-Methoxy-5-trifluormethyl-, Trimethoxy- oder 3,5-Dichlorphenyl; Thienyl-2-yl und 5-Niederalkyl-, 5-Phenyl, 4-Cyclopentyl, 5-Cyclohexyl, 5-Chlor- oder 5-Bromthien-2-yl, 5-Allylthien-2-yl;

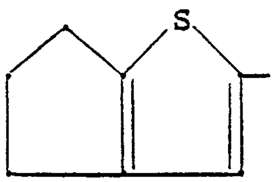 , 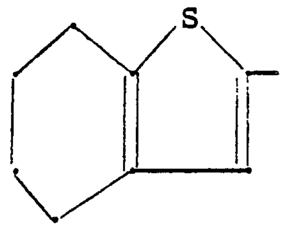 , 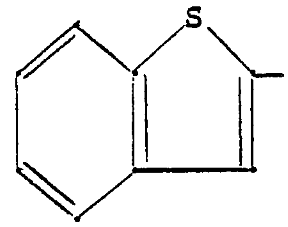

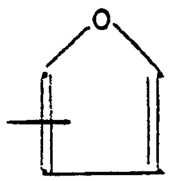 , 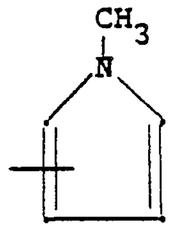 , 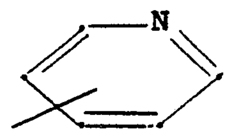 ,

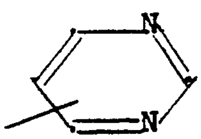 , 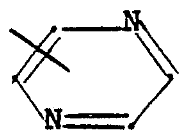 , 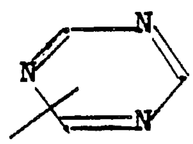 ,

wobei diese Ringsysteme gegebenenfalls auch zusätzliche Substituenten enthalten können, insbesondere Halogenatome, niedere Alkyl- und Alkoxygruppen, sowie $N(R_{16})_2$. Für $R_2$ repräsentative Gruppen sind niedere Alkyl- und Alkoxyreste, $C_3$-$C_6$-Cycloalkyle, halogensubstituierte Alkyl- und Alkoxygruppen wie $CF_3$, $OCF_3$, $C_2F_5$, $OC_2F_5$, ferner $CH_2N(R_{16})_2$ sowie etwa Methoxyethyl, Ethoxyethoxy, Allyl und Propargyl.

Die Gruppe $R_3$ kann besonders vielfältig variiert werden. Erwähnt seien einige einfachere Ringsysteme, von denen sich $R_3$ ableitet: Pyrrol, Pyrazol, N-Methylpyrrol, Imidazol, Thiazol, Triazol, Tetrazol, 1,2,4- und 1,3,4-Thiadiazol, Pyridin, Pyridazin, Pyrimidin, Pyrazol, wobei diese Ringe auch substituiert sein können, z.B. durch niedere Alkylgruppen oder Halogenatome.

Weiterhin seien erwähnt:

Phenyl, Phenylphenyl, Phenoxyphenyl, wobei diese Reste z.B. auch durch Niederalkyl, Niederalkoxy, $N(R_{16})_2$, $CF_3$, Halogen substituiert sein können; von kondensierten Systhemen abgeleitete Reste, wie Theophyllin, Benzimidazol,

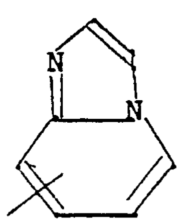 , 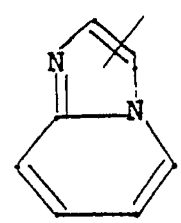 , 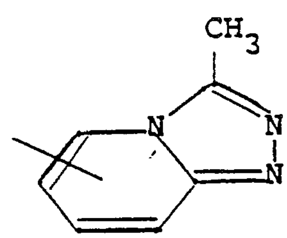

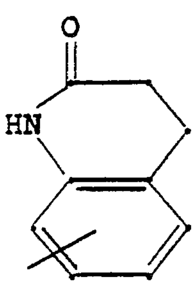 , 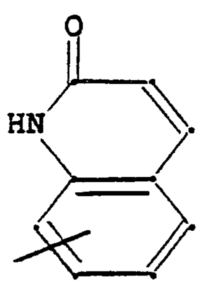 , 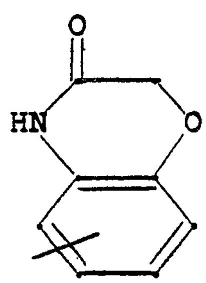 , 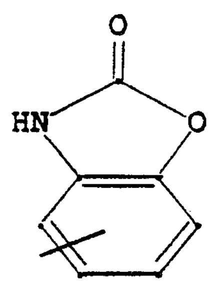

Auch die kondensierten Systeme können zusätzliche Substituenten, etwa OH, $CH_3$, $OCH_3$, Cl, enthalten.

Stellen die Gruppen II bis X′ kondensierte Ringsysteme dar, so können sie auch über den ankondensierten Ring gebunden sein.

Die neuen Verbindungen werden nach an sich bekannten Verfahren hergestellt. Solche Verfahren sind beispielsweise für die 1,2,4-Triazole beschrieben in Chemistry of Heterocyclic Compounds, Vol. 37, S. 34 ff und 66 ff., (J. A. Montgomery (ED), "1,2,4-Triazoles", New York, Willey (1981) und für die Imidazole in Rodd's Chemistry of Carbon Compounds, 2nd edition, Vol. IV, Part C, S. 119 ff, (M. F. Ansell, New York, Elsevier (1986)).

Hier seien die Verfahren näher angegeben:

1. Verbindungen der Formel

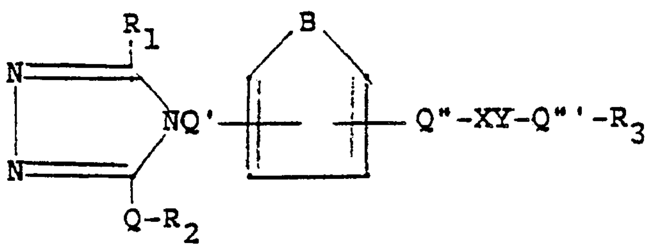

(Ia/Ib)

werden durch Erhitzen von Acylamidrazonen der Formel

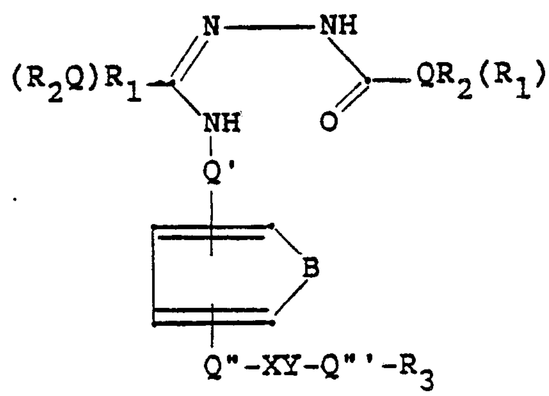

(XI),

in der $R_1$, $R_2$, $R_3$, B, Q, Q′, Q″, Q‴, X und Y die obige Bedeutung haben und, wie angedeutet, $R_1$ und $R_2Q$ hier wie in den weiteren Formeln ihre Positionen tauschen können, ohne Lösungsmittel oder in einem inerten Lösungsmittel erhalten. Die Reaktionstemperatur beträgt vorzugsweise 150 -200 °C. Als Lösungsmittel werden zweckmäßig solche verwendet, deren Siedetemperatur in dem genannten Bereich liegt, so daß die Umsetzung unter Rückfluß stattfinden kann.

Verbindungen der Formel XI können z.B. wie folgt erhalten werden:

(a) Imidsäureester (XII) werden mit Carbonsäurehydraziden (XIII) zu den Carbonsäurehydrazonidestern (XIV) umgesetzt, die mit den Aminoverbindungen (XV) Acylamidrazone ergeben.

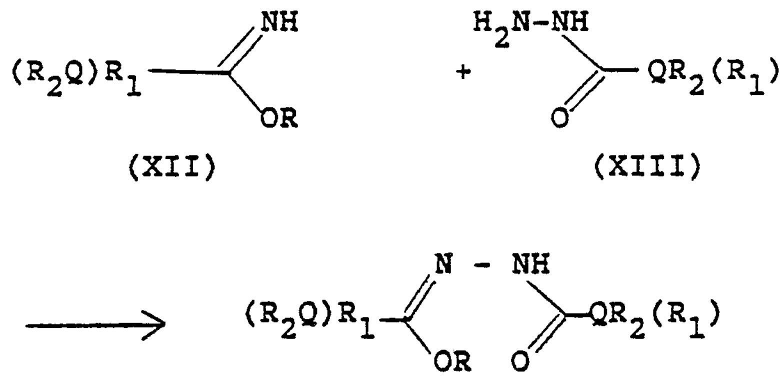

(XII) (XIII)

(XIV)

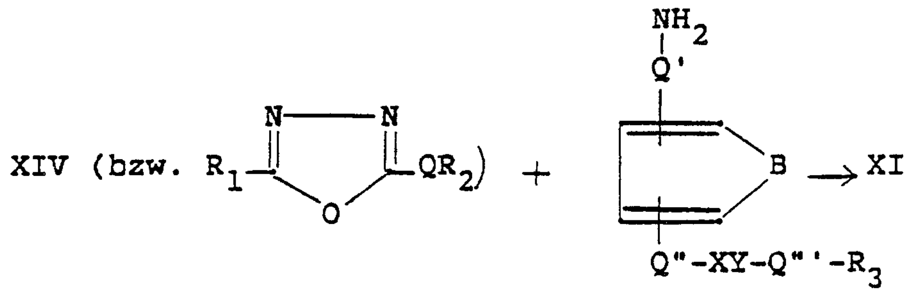


(XV)

In den vorstehenden Formeln steht R für einen niederen Alkylrest, die anderen Symbole haben die oben angegebenen Bedeutungen. $R_1$ und $R_2Q$ können, wie zuvor und im folgenden, jeweils umgekehrt angeordnet sein, d.h. anstelle von $R_1$ in den Formeln kann $R_2Q$ stehen, dann steht anstelle von $R_2Q$ entsprechend $R_1$.

(b) Entsprechende Amidrazine werden mit Carbonsäurehalogeniden, Carbonsäureanhydriden bzw. Orthoestern umgesetzt:

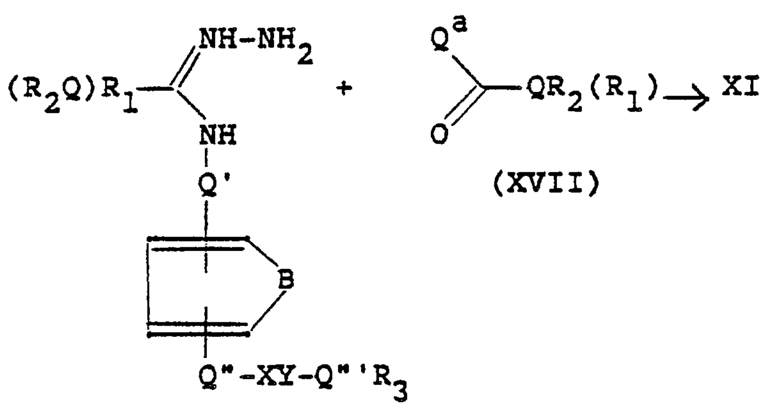


(XVII)

(XVI)

Qa = Halogen oder $-O-COQR_2$

statt XVII ist auch $(RO)_3CQR_2$ verwendbar.

(c) Thioamide bzw. S-Methylisothioamide bzw. Chlorimide werden mit Hydraziden umgesetzt:

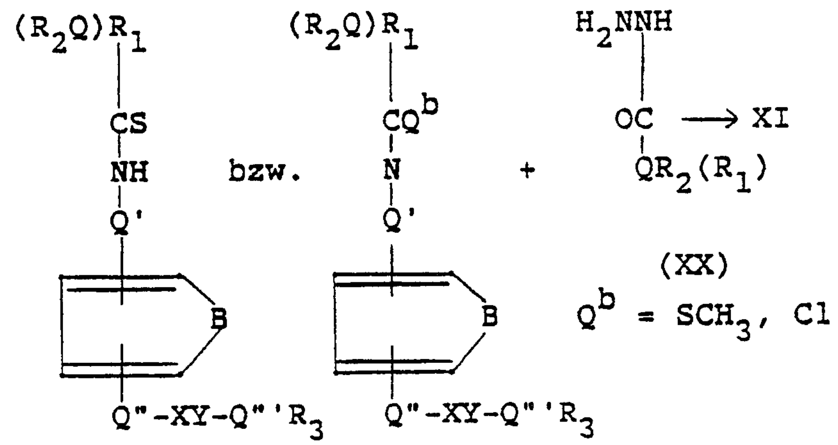


(XVIII) (XIX)

2. Verbindungen der Formel Ia bzw. Ib, in denen A für CH steht, werden durch Umsetzung von N-Chloramidinen der Formel XXI mit Enaminen bzw. Silylenolethern der Formel XXII erhalten:

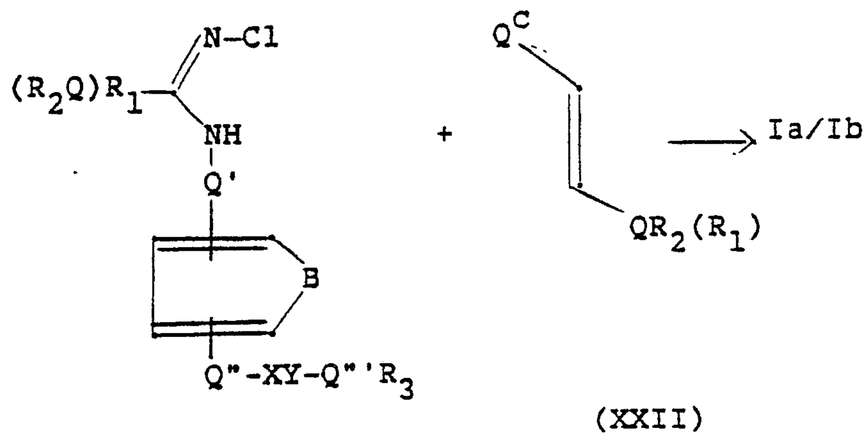


(XXII)

(XXI)

$Q^c$ = N(Niederalkyl)$_2$, OSi(Niederalkyl)$_3$
Die Umsetzung erfolgt in inerten organischen Lösungsmitteln, z.B. Chlorkohlenwasserstoffen wie Chloroform in Gegenwart einer tertiären Base, z.B. Pyridin, bei Temperaturen von 30-60° C.
Die N-Chloramidine XXI werden aus den entsprechenden Amidinen und N-Chlorsuccinimid erhalten. Die Enamine bzw. Silylenolether XXI sind aus entsprechenden Aldehyden mit Aminen bzw. Silylchloriden zugänglich.

3. Verbindungen der Formel Ia bzw. Ib, in denen A für CH steht, werden durch Umsetzung von substituierten Amiden XXIII mit Aminen der Formel XXIV in Gegenwart von $PCl_3$ in der Wärme erhalten.

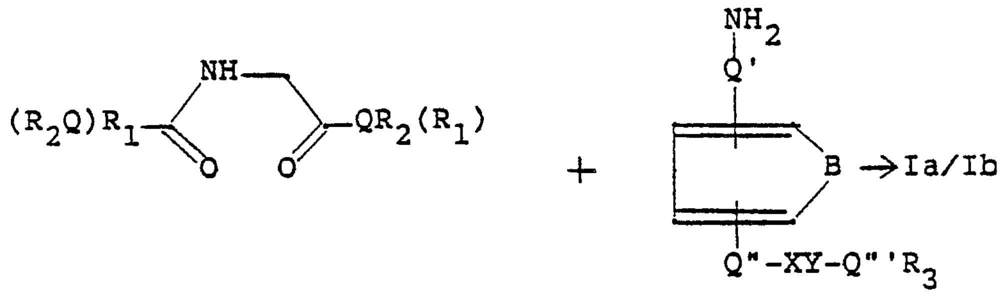

(XXIII) (XXIV)

Die Umsetzung erfolgt in inerten Lösungsmitteln wie Chlorbenzol bei etwa 100 bis 150° C. Die Amide (XXIII) sind beispielsweise aus entsprechenden Säurechloriden und α-Aminoketonen erhältlich.

4. Verbindungen der Formel Ia/Ib, in denen -X-Y-CONH, CSNH, $CO(CH_2)_{1-3}NH$, $SO_2NH$ oder $SO_2$-$(CH_2)_{1-3}NH$ bedeutet, können aus den Verbindungen XXV und Aminen XXVI erhalten werden:

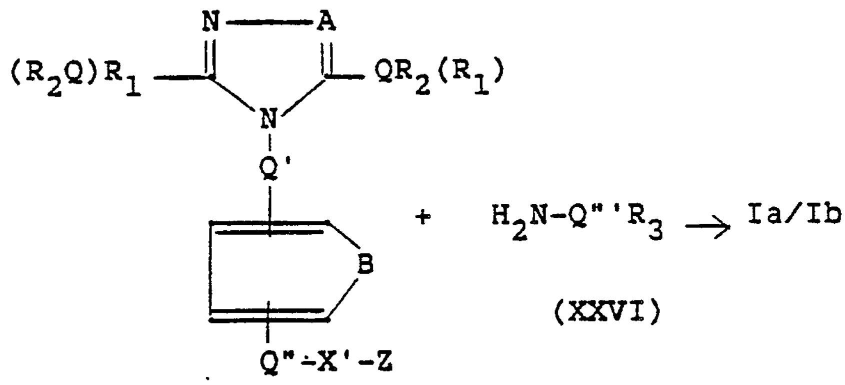

(XXVI)

(XXV)

Dabei haben A, B, $R_1$, $R_2$ und $R_3$ die obige Bedeutung, X′ steht für CO, $CO(CH_2)_{1-3}$, $SO_2$, CS oder $SO_2$-$(CH_2)_{1-3}$ und Z für OH, Halogen, Niederalkoxy, Niederalcyloxy. Die Umsetzung erfolgt unter üblichen Bedingungen, z.B. wie in Houben-Weyl VIII, 653ff und E 5, 941 ff beschrieben.

5. Zur Herstellung von Verbindungen der Formel I, in denen X-Y- NH-CO oder NH-CO-NH bedeutet, können Amine XXVII mit Carbonsäuren bzw. Carbonsäurederivaten XXVIII bzw. mit Isocyanaten XXIX nach üblichen Methoden entsprechend Verfahren 4 umgesetzt werden:

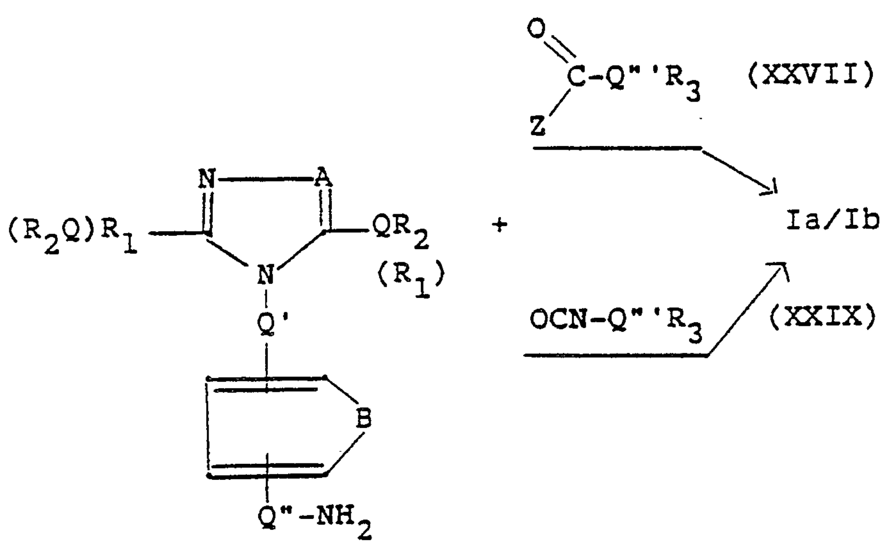

(XXVII) (XXIX)

(XXVII)

A, B, Q, Q′, Q″, Q‴, $R_1$, $R_2$, $R_3$ und Z haben dabei die oben angegebene Bedeutung.
Die Ausgangsstoffe für Verfahren 4 und 5 werden nach üblichen Methoden hergestellt, XXV und XXVII können z.B. analog den Verfahren 1 bis 3 gewonnen werden.

Gewünschtenfalls werden nach Verfahren 1 bis 5 zunächst erhaltene Basen in üblicher Weise in Säureadditionssalze überführt, zunächst erhaltene Salze in freie Basen.

Die erfindungsgemäßen Verbindungen besitzen PAF-antagonistische Wirkung.
Bekanntlich handelt es sich bei PAF (Plättchen Aktivierender Faktor) um das Phospholipid Acetyl-glyceryl-ether-phosphoryl-cholin (AGEPC), das als potenter Lipidmediator bekannt ist, der von tierischen und menschlichen proinflammatorischen Zellen freigesetzt wird. Unter solchen Zellen finden sich hauptsächlich basophile und neutrophile Granulozyten, Makrophagen (aus Blut und Gewebe) sowie Thrombozyten, die an Entzündungsreaktionen beteiligt sind.

PAF zeigt im pharmakologischen Experiment Bronchokonstriktion, Blutdrucksenkung, Auslösung einer Thrombozytenaggregation sowie eine proinflammatorische Wirkung.
Diese experimentell nachweisbaren Wirkungen des PAF weisen direkt oder indirekt auf mögliche Funktionen dieses Mediators in der Anaphylaxie, in der Pathophysiologie des Asthma bronchiale und allgemein in der Entzündung hin.

PAF-Antagonisten werden benötigt, um einerseits weitere pathophysiologische Funktionen dieses Mediators an Tier und Mensch aufzuklären und andererseits pathologische Zustände und Krankheiten, an denen PAF beteiligt ist, insbesondere entzündliche und allergische Vorgänge, zu behandeln. Beispiele für mögliche Indikationen eines PAF-Antagonisten sind Entzündungsprozesse des Tracheobronchialbaumes (akute und chronische Bronchitis, Asthma bronchiale) oder der Niere (Glomerulonephritis), der Gelenke (rheumatische Erkrankungen), anaphylaktische Zustände, Allergien und Entzündungen im Bereich der Schleimhäute (Rhinitis, Konjunktivitis) und der Haut (z.B. Psoriasis) sowie durch Sepsis, Endotoxine oder Verbrennungen bedingte Schockzustände. Weitere wichtige Indikationen für einen PAF-Antagonisten sind Läsionen und Entzündungen im Bereich der Magen- und Darmschleimhaut, wie z.B. Schockulcus, Colitisulcerose, Morbus Crohn, Stressulcus, im allgemeinen Ulcus pepticum, jedoch insbesondere Ulcus ventriculi und Ulcus duodeni;
Obstruktive Lungenerkrankungen, wie z.B. bronchiale Hyperreaktivität, entzündliche Lungenwegserkrankungen, wie z.B. chronische Bronchitis;
Herz- Kreislauferkrankungen, wie z.B. Polytrauma, Anaphylaxe, Arteriosklerose, entzündliche Darmerkrankungen, EPH-Gestose (ederma-proteinuria Hypertension), Erkrankungen des extrakorporalen Kreislauf z.B. Herzinsuffizienz, Herzinfarkt, Organschäden bei Bluthochdruck, ischämische Erkrankungen, entzündliche und immunologische Erkrankungen, Immunmodulation bei Transplantationen von Fremdgeweben, etwas die Abstoßung von Nieren-, Leber- und anderen Transplantaten, Immunmodulation bei Leukämie. Metastasenausbreitung (z.B. bei bronchialer Neoplasie), Erkrankungen des ZNS, wie z.B. Migräne, multiple Sklerose, endogene Despression, Agarophobie (panic disorder), weiterhin erweisen sich die erfindungsgemäßen Verbindungen als Cyto- und Organoprotektion z.B. zur Neuroprotektion, etwa bei Leberzirrhose, DIC (disiminierte intravasale Gerinnung);
Nebenwirkungen einer Arzneimitteltherapie, z.B. anaphylaktoide Kreislaufreaktionen, Kontrastmittelzwischenfälle, Nebenwirkungen bei der Tumortherapie;
Unverträglichkeiten bei Bluttransfusionen; fulminantes Leberversagen ($CCl_4$-Intoxikation) Amanitaphalloides-Intoxikation (Knollenblätterpilzvergiftung);
Symptome von parasitären Erkrankungen (z.B. Wurmerkrankungen); Autoimmunerkrankungen (z.B. M. Werlhof); Autoimmunhaemolytischen Anaemien, autoimmunologisch bedingte Glomerulonephritiden, Thyreoidis Hashimoto, primäres Myxoedem, perniziöse Anaemie, autoimmune atrophische Gastritis, Morbus Addison, iuveniler Diabetes, Goodpasture-Syndrom, idiopathische Leukopenie, primär biliäre Zirrhose, aktive bzw. chronisch aggressive Hepatitis (HBsAg-neg.), Colitis ulcerosa und systemischer Lupus erythematodes (SLE), ideopathische thrombozytopenische Parpura (ITP).

Immunfunktion bei Aids, Diabetes, juvenile Diabetes, diabetische Retinopathie, polytraumatischer Schock, hämorrhagischer Schock; PAF-assoziierte Interaktion mit Gewebshormonen (autocoid hormones), Lymphokinen und anderen Mediatoren.

Sie können auch in Kombinationen eingesetzt werden, für die PAF-Antagonisten geeignet sind, etwa mit ß-Adrenergika, Parasympatholytika, Corticosteroide, Antiallergika, Sekretolytika. Bei der Kombination mit TNF (Tumor-Nekrose-Faktor) wird eine bessere Verträglichkeit (Ausschaltung störender Nebenwirkungen) des TNF erreicht; TNF läßt sich daher gewünschtenfalls auch in höheren Dosen einsetzen als bei seiner alleinigen Anwendung. (Unter "Kombination" ist hier auch die Anwendung der beiden Wirkstoffe in getrennten Zubereitungen und in einem gewissen zeitlichen Abstand zu verstehen). Bei der gemeinsamen

Anwendung der erfindungsgemäßen Verbindungen mit ß-Adrenergika kann ein synergistischer Effekt erzielt werden, z.B. in der Broncholyse.

Als Maß für die PAF-antagonistische Wirkung seien die Hemmkonzentrationen ($IC_{50}$) bei der PAF-induzierten Thrombocytenaggregation angegeben:

Verbindungen der Formel

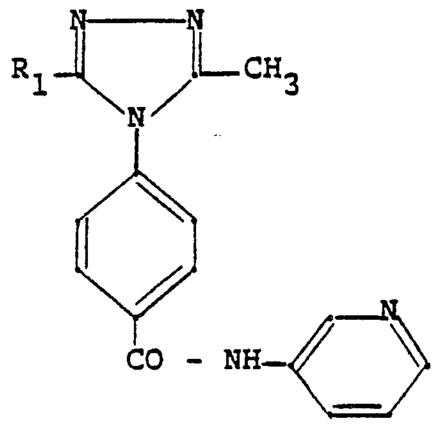

| Nr. | $R_1$ | $IC_{50} \times 10^{-6}M$ |
|---|---|---|
| 1 | 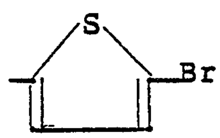 | 0,99 |
| 2 | 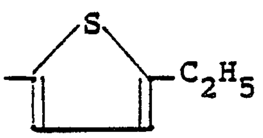 | 0,61 |
| 3 | 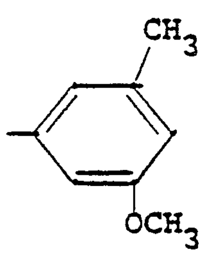 | 0,13 |

Die neuen Verbindungen können topisch, oral, transdermal, parenteral oder durch Inhalation verabreicht werden. Die Verbindungen liegen hierbei als aktive Bestandteile in üblichen Darreichungsformen vor, z.B. in Zusammensetzungen, die im wesentlichen aus einem inerten pharmazeutischen Träger und einer effektiven Dosis des Wirkstoffes bestehen, wie z.B. Tabletten, Dragées, Kapseln, Oblaten, Pulver, Lösungen, Suspensionen, Inhalationsaerosole, Salben, Emulsionen, Sirupe, Suppositorien.

Die therapeutische und prophylaktische Dosis ist abhängig von der Beschaffenheit und Ernsthaftigkeit des Krankheitszustandes.

Eine wirksame Dosis der erfindungsgemäßen Verbindungen liegt bei oraler Anwendung zwischen 1 und 100, vorzugsweise zwischen 10 und 80 mg/Dosis, bei intravenöser oder intramuskulärer Anwendung zwischen 0,001 und 50, vorzugsweise zwischen 0,1 und 30 mg/Dosis. Für die Inhalation sollen Lösungen, die 0,01 bis 1,0, vorzugsweise 0,1 bis 0,5 % Wirkstoff enthalten, eingesetzt werden, ferner Pulver und

Suspensionen in verflüssigten Treibgasen.

Formulierungsbeispiele

1. Tabletten

| Zusammensetzung: | |
|---|---|
| Wirkstoff gemäß der Erfindung | 30 Gew.-Teile |
| Stearinsäure | 6 Gew.-Teile |
| Traubenzucker | 564 Gew.-Teile |

Die Bestandteile werden in üblicher Weise zu Tabletten von 600 mg Gewicht verarbeitet. Gewünschtenfalls kann der Wirkstoffgehalt erhöht oder vermindert und die Traubenzuckermenge entsprechend vermindert oder erhöht werden.

2. Suppositorien

| Zusammensetzung: | |
|---|---|
| Wirkstoff gemäß der Erfindung | 80 Gew.-Teile |
| Laktose, gepulvert | 45 Gew.-Teile |
| Kakao-Butter | 1575 Gew.-Teile |

Die Bestandteile werden in üblicher Weise zu Suppositorien von 1,7 g Gewicht verarbeitet.

3. Inhalationspulver

Mikronisiertes Wirkstoffpulver (Verbindung der Formel I; Teilchengröße ca. 0,5 bis 7 mm) werden in einer Menge von 0,02 mg mit 10 mg mikronisierter Lactose und gegebenenfalls geeigneten Mengen weiterer Wirkstoffe in Hartgelatinekapseln abgefüllt. Das Pulver wird aus üblichen Inhalationsgeräten, z.B. gemäß DE-A 3345 722, inhaliert.

4. Dosieraerosol

| | |
|---|---|
| Wirkstoff gemäß der Erfindung | 0,5 Gew.-% |
| Sorbitantrioleat | 0,5 Gew.-% |
| Monofluortrichlormethan und Difluordichlormethan (2:3) | 99,0 Gew.-% |

Die Mischung wird in Dosieraerosolgeräte üblicher Art abgefüllt. Die Dosiervorrichtung wird beispielsweise so ausgelegt, daß pro Hub 0,05 ml der Zubereitung abgegeben werden.

Die unten beschriebenen Beispiele sollen die Verfahren näher erläutern.

Beispiel 1

3-Methyl-5-(5-methyl-2-thienyl)-4-[4-[N-(3-pyridyl)-carbamoyl]-phenyl]-4H-1,2,4-triazol

2,9 g Essigsäure-[2-(5-methyl-2-thenoyl)-hydrazonid]ethylester, Fp. 110-113° C, (hergestellt aus 5-Methylthiophencarbonsäurehydrazid, Fp. 102-104° C, durch Umsetzung mit Acetimidsäurethylesterhydrochlorid) und 2,1 g p-Aminobenzoesäure-N-(3-pyridyl)-amid, Fp. 200-202° C (hergestellt aus p-Nitrobenzoylchlorid durch Umsetzen mit 3-Aminopyridin und katalytischer Reduktion der Nitroverbindung (Fp. 182-184° C), mit Raney-Nickel) werden 2 Stunden auf 170-190° C erwärmt. Der ölige Rückstand wird in 150 ml Chloroform durch Erwärmen gelöst, die unlöslichen Bestandteile werden abfiltriert, die Chloroformphase mit verdünnter Essigsäure und verdünnter Ammoniaklösung gewaschen, getrocknet und abdestilliert. Der Rückstand wird in 20 ml Essigester gelöst, abgekühlt, die ausgefallene Titelverbindung (1,2 g; 32 % d.Th.) wird abgesaugt und aus Ethanol umkristallisiert;
Fp. 206-208° C.

Beispiel 2

3-Methyl-5-phenyl-4-[4-[N-(3-pyridyl)- carbamoyl]-phenyl]-4H-1,2,4-triazol

1,7 g 4-(4-Carboxyphenyl)-3-methyl-5-phenyl-4H-1,2,4-triazol, Fp. > 270° C (hergestellt aus Essigsäure-(2-phenylhydrazonid)-ethylester durch Umsetzen mit p-Aminobenzoesäureethylester und Verseifung des Esters (Fp. 140-142° C), werden in 6,3 ml Dimethylformamid und 25 ml Tetrahydrofuran aufgeschlämmt und mit 0,91 ml Triethylamin versetzt. Die Lösung wird auf 10° C abgekühlt und unter Rühren wird eine Mischung von 2,5 ml Tetrahydrofuran / 10,59 ml Chlorameisensäurethylester zugetropft. Nach 30 Minuten wird das ausgefallene Triethylamin-hydrochlorid abgesaugt, mit 5 ml Tetrahydrofuran gewaschen und die Lösung mit 0,58 g 3-Aminopyridin gelöst in 5 ml Tetrahydrofuran versetzt. Anschließend wird das Tetrahydrofuran abdestilliert, der Rückstand eine Stunde auf 80-90° C erhitzt, das Dimethylformamid wird abdestilliert, der Rückstand in 100 ml Chloroform gelöst, einmal mit Wasser gewaschen, getrocknet und abdestilliert. Der Rückstand wird in 10 ml Essigester gelöst, die ausgefallene Titelverbindung (1 g; 46 % d.Th.) wird abgesaugt und aus Acetonitril umkristallisiert (Fp. 194-196° C).

Beispiel 3

3-Methyl-4-[4-(1-imidazolyl)-acetamidophenyl]-5-(2-thienyl)-4H-1,2,4-triazol

6,4 g 4-(4-Aminophenyl)-3-methyl-5-(2-thienyl)-4H-1,2,4-triazol (Fp. 226-8° C), [hergestellt aus Essigsäure[2-(3-thenoyl)-hydrazonid]ethylester durch Umsetzen mit 4-Aminoacetanilid und Verseifung des Amids (Fp. 210-2° C)], werden in 100 ml Ethylenchlorid unter Zusatz von 2,2 ml Pyridin mit 2,1 ml Chloracetylchlorid umgesetzt.

3,6 g Chloracetylverbindung (Fp. >280° C) werden in eine Mischung von 25 ml Dimethylformamid, 0,75 g Imidazol, 0,55 g Natriumhydrid Dispersion (ca. 55%ig) portionsweise eingetragen und 2 Stunden bei 80-100° C gerührt. Die Lösung wird dann mit Wasser verdünnt, 30 Minuten unter Zusatz vvon 20 ml Essigester gerührt. Die ausgefallenen Kristalle werden abgesaugt und aus Methanol umkristallisiert.
Ausbeute 2,5 g (61 % d.Th.);
Fp. 190-193° C (Hydrat).

Beispiel 4

1-[4-(N-pyridyl)carbamoyl]-2-methyl-5-(3,5-dimethoxyphenvl)-imidazol

2 g 1-(4-Carbethoxyphenyl)-2-methyl-5-(3,5-dimethoxyphenyl)-imidazol [hergestellt aus 3,5-Dimethoxy-α-acetaminoacetophenon durch Umsetzen mit 4-Aminobenzoesäureethylester] werden in 50 ml Ethanol und 20 ml l0%iger Natronlauge zur Säure verseift. Anschließend wird die Säure in 500 ml Chloroform und 2 ml Thionylchlorid 2 Stunden am Rückfluß gekocht, das Chloroform abdestilliert und das Säurechlorid in 50 ml Dioxan mit einer Lösung aus 2,5 g 3-Aminopyridin in 50 ml Dioxan versetzt. Die Lösung wird 30 Minuten auf 80° C erhitzt, danach mit 200 ml Eiswasser verdünnt. Das Produkt wird mit Chloroform extrahiert und

über eine Kieselgel-Säule gereinigt (Eluens Chloroform/Ethanol 8:2).
Man erhält die Titelverbindung in einer Ausbeute von 0,6 g (26,5 %d.Th.);
Fp. 179-81 °C (aus Chloroform/Petroläther).

Beispiel 5

4-[3-(3,5-Dimethoxyphenyl)-5-methyl-4H-1,2,4-triazol-4-yl]-N-(3-pyridyl)benzamid

3,5-Dimethoxybenzoesäure wird in Chloroform bei Rückflußtemperatur mit Thionylchlorid in das Säurechlorid übergeführt und dieses mit 4-Aminobenzoesäureethylester in Gegenwart von Triethylamin in der Wärme zum Amid umgesetzt, Fp. 133-5 °C. Das Amid wird in Toluol unter Ruckfluß mit Phosphorpentachlorid umgesetzt. Das Reaktionsprodukt wird in Toluol mit Acethydrazid und Triethylamin unter Rückfluß gekocht. Man isoliert das Reaktionsprodukt, löst es in Methanol, versetzt mit verdünnter wäßriger Natronlauge und erhitzt 30 Minuten unter Rückfluß. Die Lösung wird angesäuert, das Reaktionsprodukt isoliert und aus Dimethylformamid umkristallisiert; Fp. >250 °C.

Eine Mischung aus 4,25 g der erhaltenen Säure in 70 ml Dimethylformamid und Tetrahydrofuran (1:1) und 2 g Triethylamin wird vorgelegt, überschüssiger Chlorameisensäureethylester in Tetrahydrofuran wird zu der gekühlten Mischung zugetropft und noch ca. 1 Stunde gerührt. Man saugt das ausgefallene Triethylaminhydrochlorid ab, versetzt die Lösung mit 1,5 g 3-Aminopyridin und erhitzt etwa 1 Stunde zum Ruckfluß. Nach dem Entfernen der Lösungsmittel erhält man einen öligen Rückstand, der in Chloroform aufgenommen wird. Man wäscht die Chloroformphase mit verdünnter Natronlauge und verdünnter Essigsäure, trocknet mit Natriumsulfat und destilliert das Lösungsmittel weitgehend ab. Beim Abkühlen erhält man Kristalle, die nach weiterer Reinigung den Fp. 245 °C zeigen; Ausbeute ca. 50 % d.Th.

Entsprechend den vorstehenden Beispielen können auch die Verbindungen der folgenden Tabellen erhalten werden:

## TABELLE I

Verbindungen der Formel

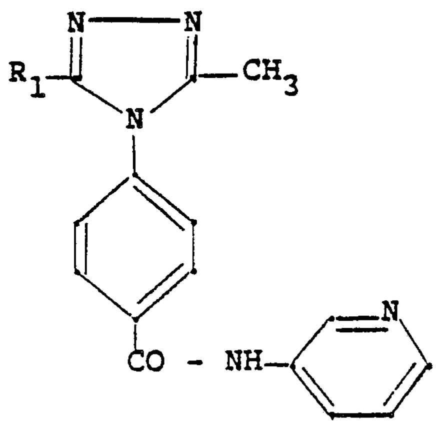

| Nr. | $R_1$ | Fp. [°C] Base | Salz |
|---|---|---|---|
| 1 | 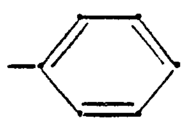 | | |
| 2 | 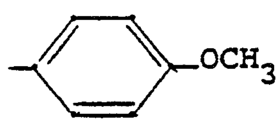 | | |
| 3 | 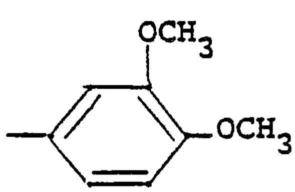 | 236-8 | |

| Nr. | $R_1$ | Fp. [°C] Base | Salz |
|---|---|---|---|
| 4 | 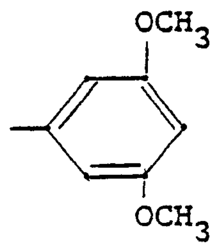 | 241-3 | |
| 5 | 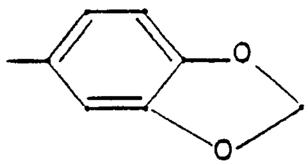 | | |
| 6 | 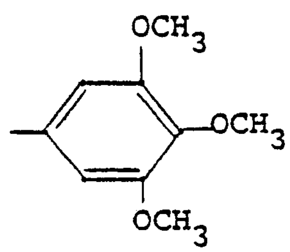 | | |
| 7 | 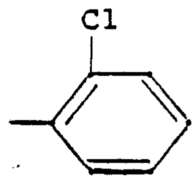 | | |
| 8 | 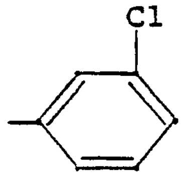 | 183-6 | |
| 9 | 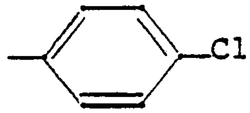 | | |

| Nr. | $R_1$ | Fp. [°C] Base | Salz |
|---|---|---|---|
| 10 | 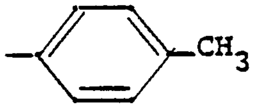 | | |
| 11 | 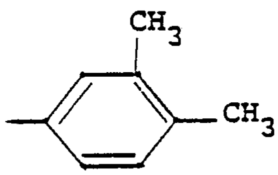 | 209-11 | |
| 12 | 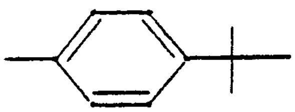 | | |
| 13 | 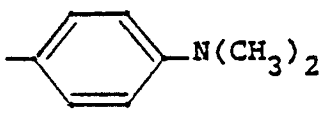 | 275-8 | |
| 14 | 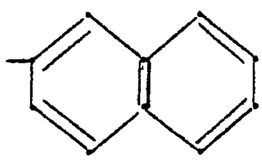 | 130 (x $CH_3OH$) | |
| 15 | 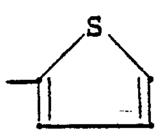 | 173-5 | |
| 16 | 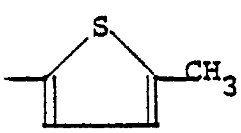 | | |
| 17 | 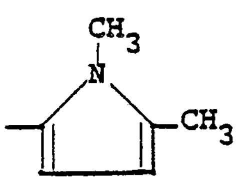 | 217 (x $H_2O$) | |

| Nr. | $R_1$ | Fp. [°C] Base | Salz |
|---|---|---|---|
| 18 | 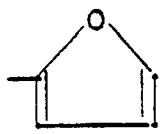 | | |
| 19 | 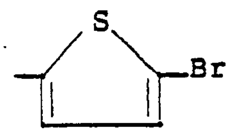 | 178-80 | |
| 20 | 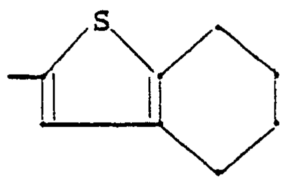 | › 280 | |
| 21 | 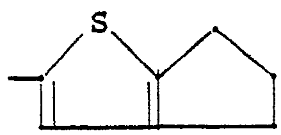 | | |
| 22 | 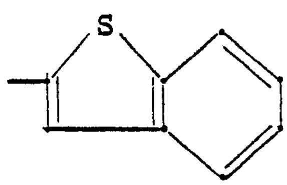 | ›260 | |
| 23 | 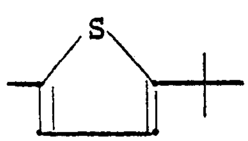 | 267 | |
| 24 | 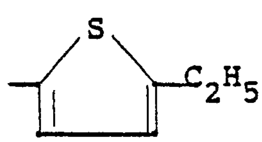 | 220 | |

| Nr. | $R_1$ | Fp. [°C] Base | Salz |
|---|---|---|---|
| 25 | 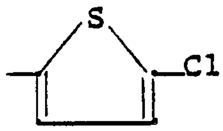 | 179-81 | |
| 26 | 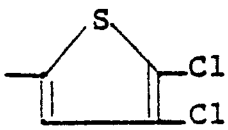 | | |
| 27 | 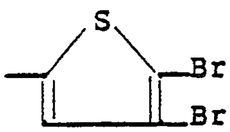 | | |
| 28 | 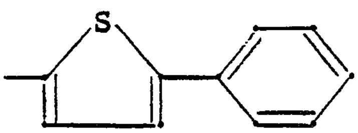 | | |
| 29 | 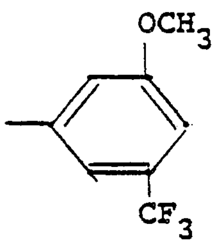 | 200-3 (x 1/2 $CH_3OH$) | |
| 30 | 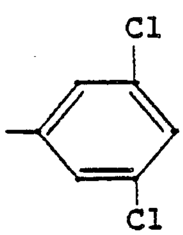 | 230-2 | |
| 31 | 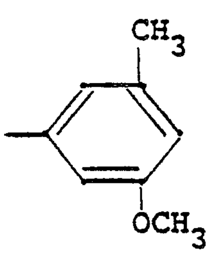 | 203-5 | |

| Nr. | $R_1$ | Fp. [°C] Base | Salz |
|---|---|---|---|
| 32 | 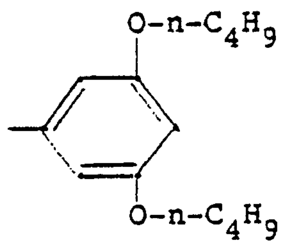 | 106-10 (x $H_2O$) | |
| 33 | 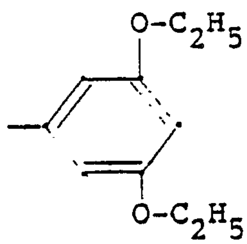 | 225-7 | |
| 34 | 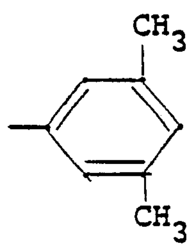 | 220-3 | |
| 35 | 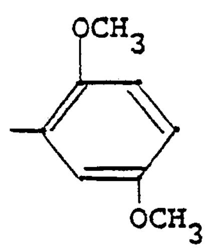 | 125-30 | |
| 36 | 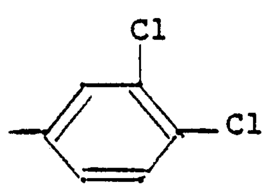 | 238-8 (x 1/2 $CH_3OH$) | |
| 37 | 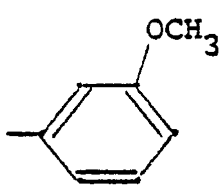 | 174-5 | |

| Nr. | $R_1$ | Fp. [°C] Base | Salz |
|---|---|---|---|
| 38 | 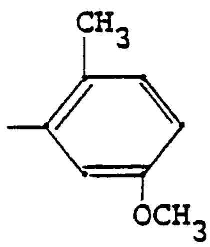 | 250-2 | |
| 39 | 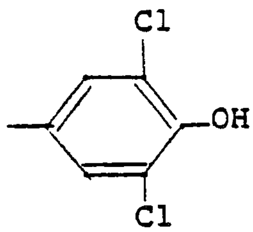 | >260 (x $CH_3OH$) | |
| 40 | 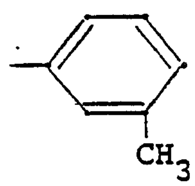 | 109-10 (x $C_2H_5OH$) | |

## TABELLE II

Verbindungen der Formel

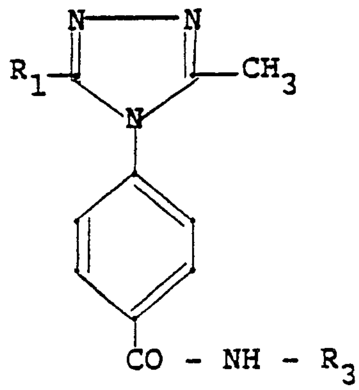

| Nr. | $R_1$ | $R_3$ | Fp.[°C] Base | Salz |
|---|---|---|---|---|
| 1 | 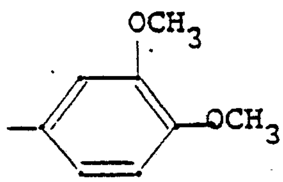 | 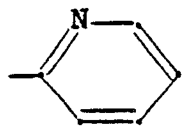 | | |
| 2 | 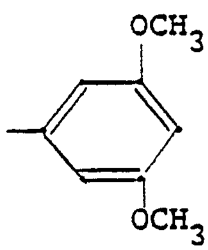 | 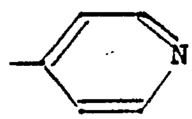 | | |
| 3 | 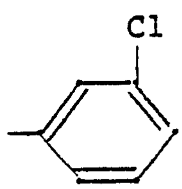 | 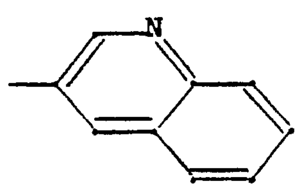 | | |

| Nr. | $R_1$ | $R_3$ | Fp.[°C] Base | Salz |
|---|---|---|---|---|
| 4 | 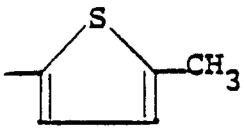 | 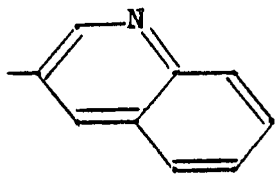 | 257-9 | |
| 5 | 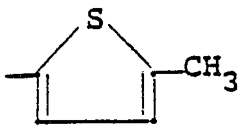 | 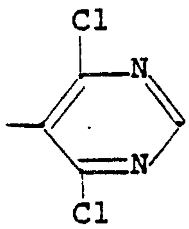 | | |
| 6 | 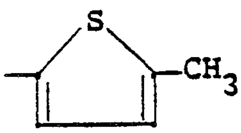 | 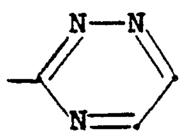 | | |
| 7 | 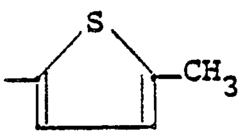 | 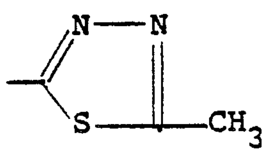 | | |
| 8 | 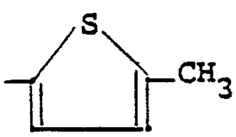 | 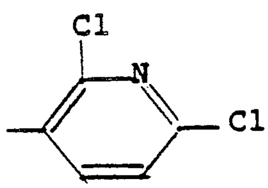 | | |
| 9 | 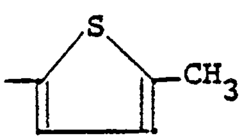 | 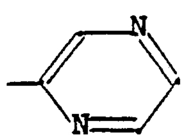 | 221-4 | |
| 10 | 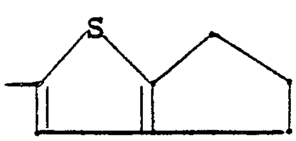 | 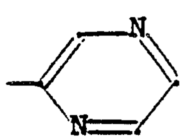 | | |

| Nr. | $R_1$ | $R_3$ | Fp.[°C] Base | Salz |
|---|---|---|---|---|
| 11 | 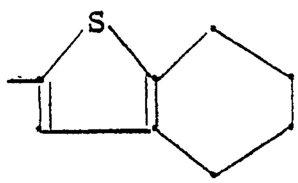 | 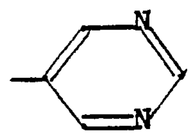 | | |
| 12 | 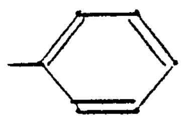 | 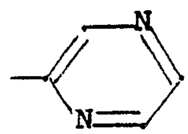 | | |
| 13 | 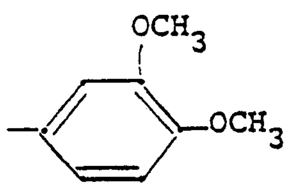 | 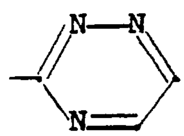 | | |
| 14 | 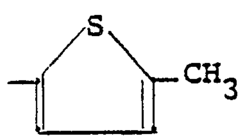 | 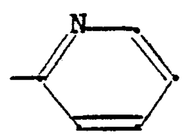 | 222-4 | |
| 15 | 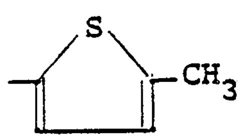 | 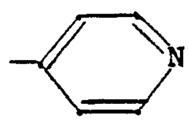 | 245-7 | |
| 16 | 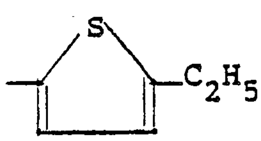 | 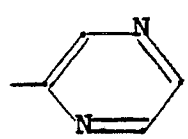 | | |
| 17 | 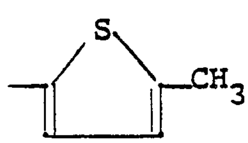 | 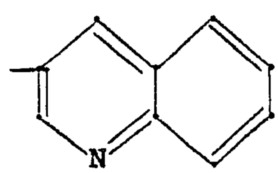 | 261-3 | |

| Nr. | $R_1$ | $R_3$ | Fp. [°C] Base | Salz |
|---|---|---|---|---|
| 18 | 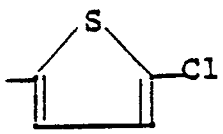 | 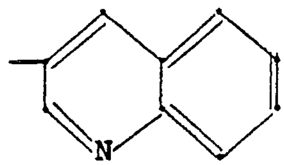 | | |
| 19 | 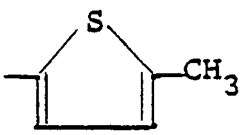 | 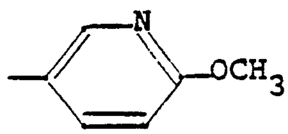 | 232-4 | |
| 20 | 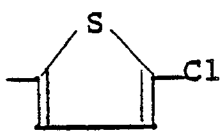 | 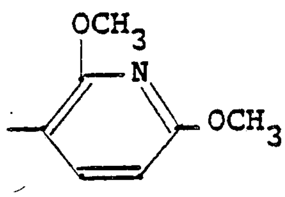 | | |
| 21 | 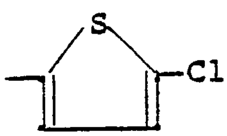 | 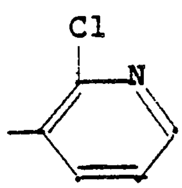 | | |
| 22 | 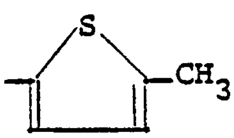 | 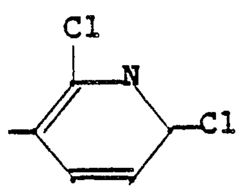 | | |
| 23 | 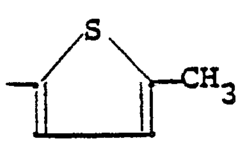 | 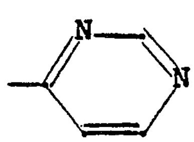 | | |
| 24 | 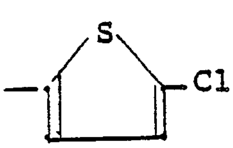 | 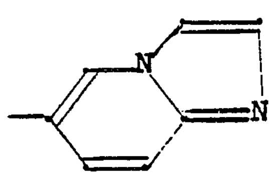 | | |

| Nr. | $R_1$ | $R_3$ | Fp.[°C] Base | Salz |
|---|---|---|---|---|
| 25 | 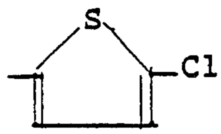 | 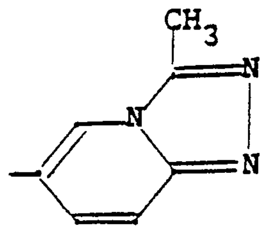 | | |
| 26 | 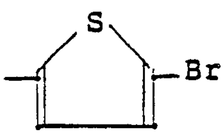 | 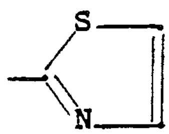 | | |
| 27 | 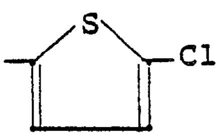 | 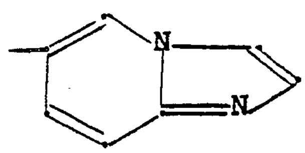 | | |
| 28 | 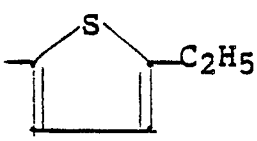 | 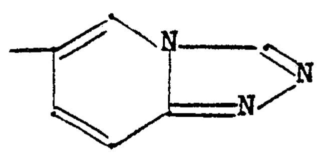 | | |
| 29 | 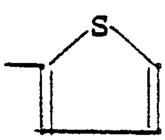 | 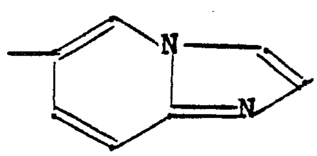 | | |
| 30 | 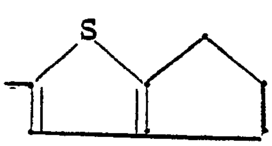 | 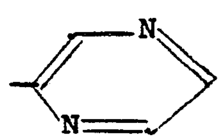 | | |
| 31 | 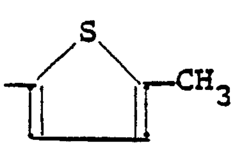 | 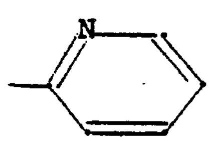 | 222-4 | |

| Nr. | $R_1$ | $R_3$ | Fp.[°C] Base Salz |
|---|---|---|---|
| 32 | 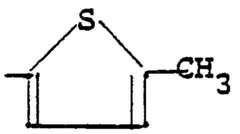 | 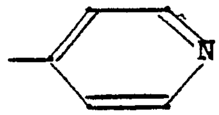 | 245-7 |
| 33 | 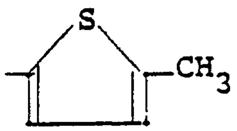 | 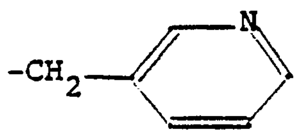 | 100-3 |
| 34 | 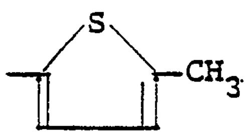 | 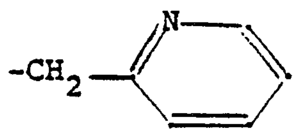 | |
| 35 | 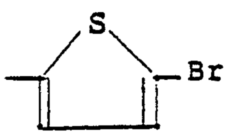 | 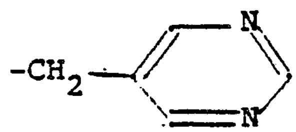 | |
| 36 | 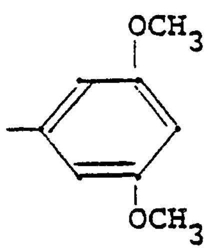 | 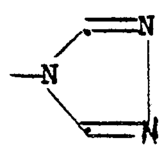 | >260 (x 1/2 $H_2O$) |
| 37 | 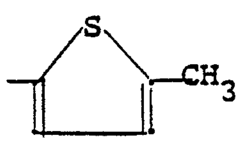 | 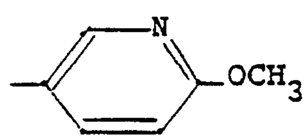 | 232-4 |
| 38 | 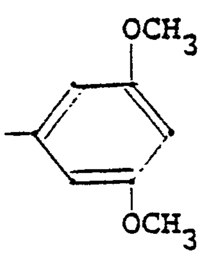 | 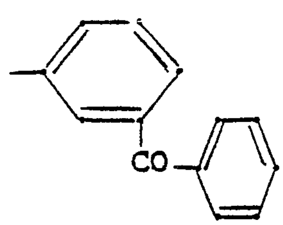 | (amorph) |

## TABELLE III

Verbindungen der Formel

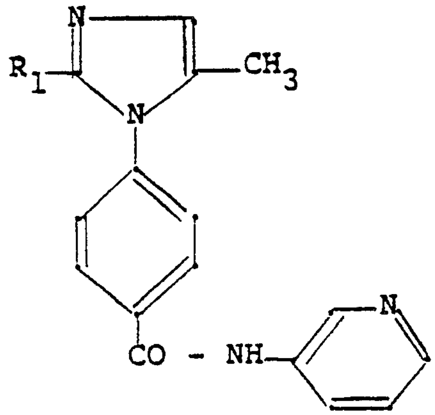

| Nr. | $R_1$ | Fp. [°C] Base | Fp. [°C] Salz |
|---|---|---|---|
| 1 | 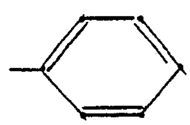 | 176 | |
| 2 | 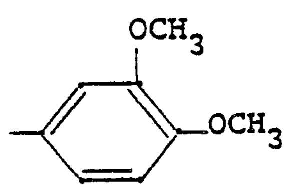 | | |
| 3 | 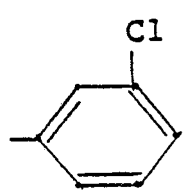 | | |

| Nr. | $R_1$ | Fp. [°C] Base | Salz |
|---|---|---|---|
| 4 | 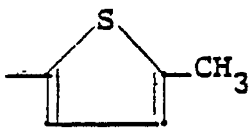 | 55 | |
| 5 | 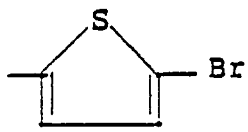 | | |
| 6 | 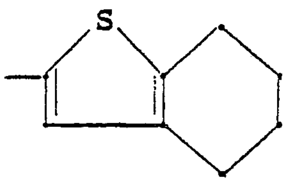 | | |
| 7 | 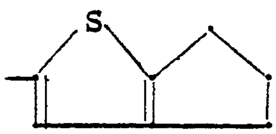 | | |
| 8 | 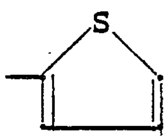 | | |
| 9 | 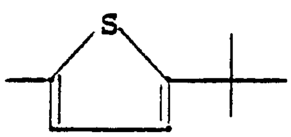 | | |
| 10 | 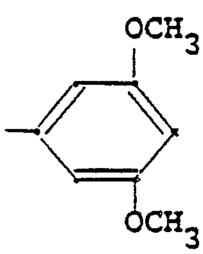 | 110 | |

| Nr. | $R_1$ | Fp. [°C] Base | Salz |
|---|---|---|---|
| 11 | 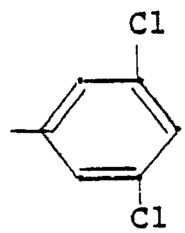 | 94-8 (x $C_2H_5OH$) | |

## TABELLE IV

Verbindungen der Formel

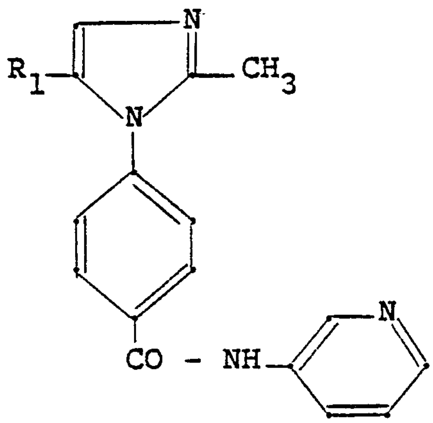

| Nr. | $R_1$ | Fp. [°C] Base | Fp. [°C] Salz |
|---|---|---|---|
| 1 | 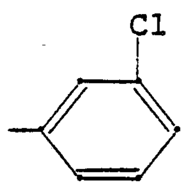 | | |
| 2 | 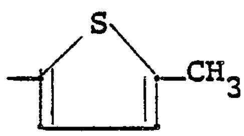 | | |
| 3 | 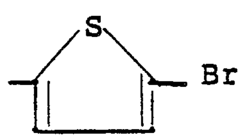 | | |
| 4 | 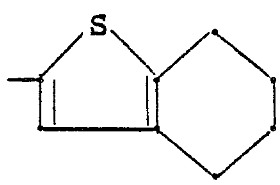 | | |

| Nr. | $R_1$ | Fp. [°C] Base | Salz |
|---|---|---|---|
| 5 | 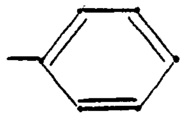 | | |
| 6 | 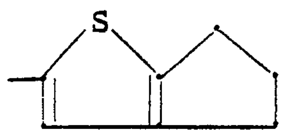 | | |
| 7 | 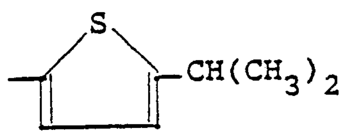 | | |
| 8 | 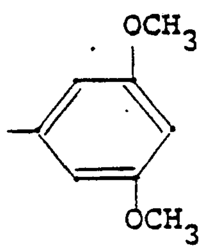 | 179-81. | |
| 9 | 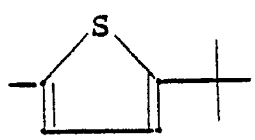 | | |
| 18 | 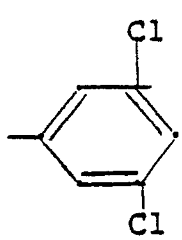 | 187-90 | |

## TABELLE V

Verbindungen der Formel

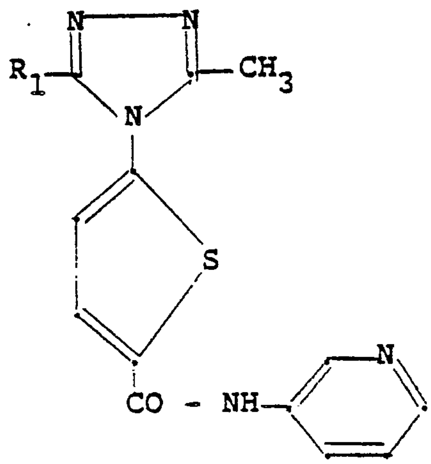

| Nr. | $R_1$ | Fp. [°C] Base | Salz |
|---|---|---|---|
| 1 | 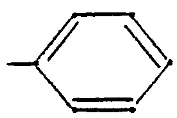 | | |
| 2 | 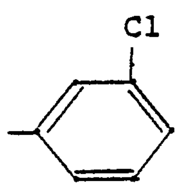 | | |
| 3 | 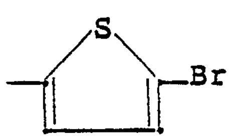 | | |
| 4 | 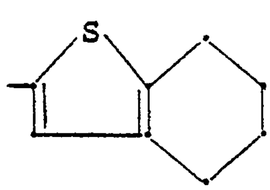 | | |

| Nr. | $R_1$ | Fp. [°C] Base | Salz |
|---|---|---|---|
| 5 | 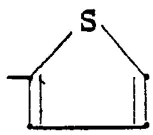 | | |
| 6 | 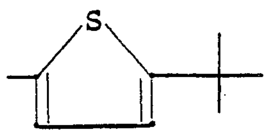 | | |
| 7 | 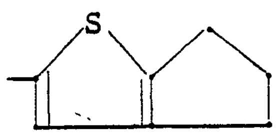 | | |
| 8 | 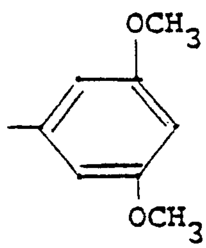 | | |
| 9 | 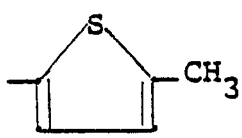 | | |

## TABELLE VI

Verbindungen der Formel

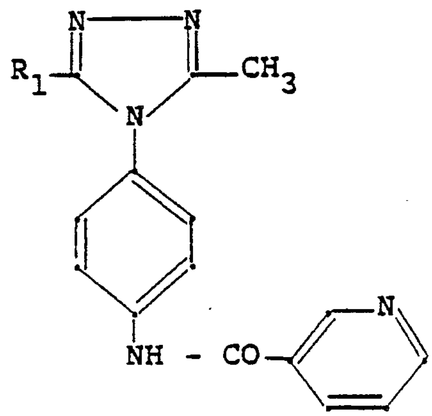

| Nr. | $R_1$ | Fp. [°C] Base | Salz |
|---|---|---|---|
| 1 | 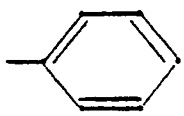 | | |
| 2 | 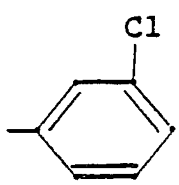 | | |
| 3 | 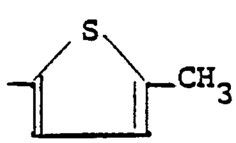 | | |
| 4 | 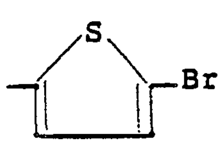 | | |
| 5 | 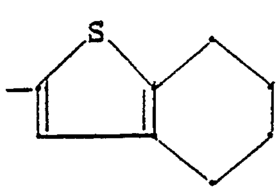 | | |

## TABELLE VII

Verbindungen der Formel

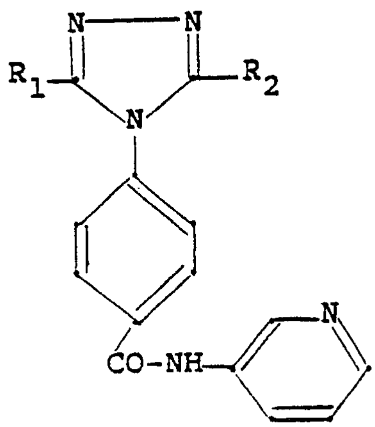

| Nr. | $R_1$ | $R_2$ | Fp[°C] |
|---|---|---|---|
| 1 | 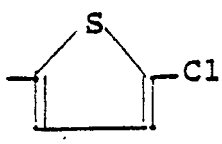 | $CF_3$ | |
| 2 | 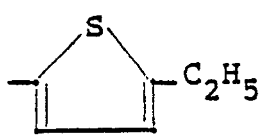 | $C_2H_5$ | |
| 3 | 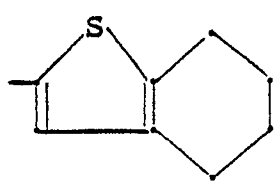 | $i\text{-}C_3H_7$ | |
| 4 | 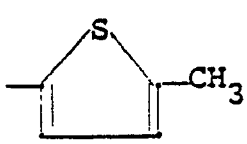 | 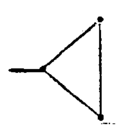 | |

| Nr. | $R_1$ | $R_2$ | Fp[°C] |
|---|---|---|---|
| 5 | 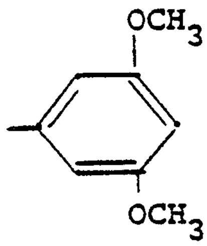 | 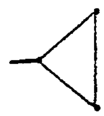 | 250-4 |
| 6 | 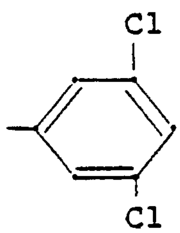 | $-i-C_3H_7$ | 194-6 |
| 7 | 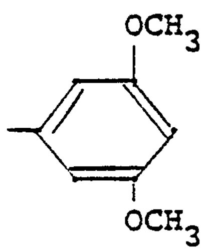 | 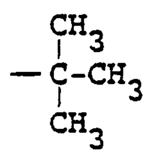 | 210 (x $H_2O$) |
| 8 | 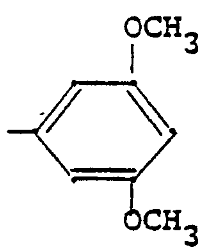 | $C_2H_5$ | 200-2 (x 1/2 $CH_3OH$) |
| 9 | 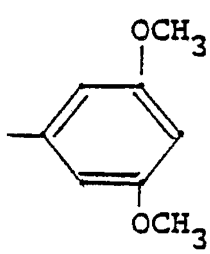 | $i-C_3H_7$ | 119-21 (x $H_2O$) |

## TABELLE VIII

Verbindungen der Formel

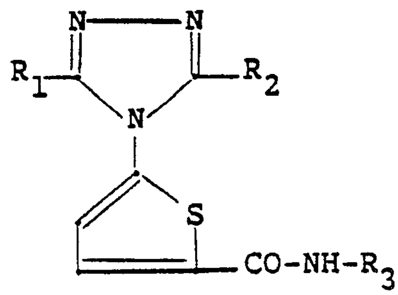

| Nr. | $R_1$ | $R_2$ | $R_3$ | Fp[°C] |
|---|---|---|---|---|
| 1 | 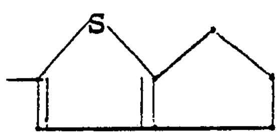 | $i-C_3H_7$ | 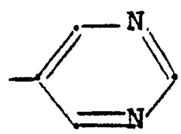 | |
| 2 | 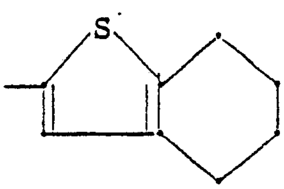 | 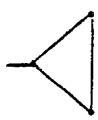 | 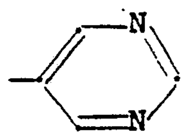 | |
| 3 | 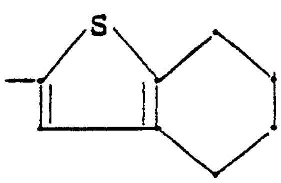 | 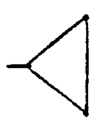 | 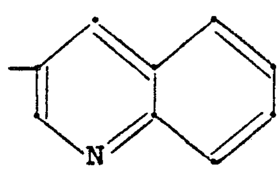 | |
| 4 | 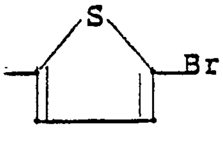 | $i-C_3H_7$ | 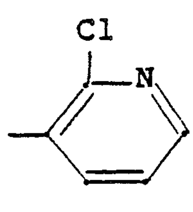 | |

| Nr. | $R_1$ | $R_2$ | $R_3$ | Fp[°C] |
|---|---|---|---|---|
| 5 | 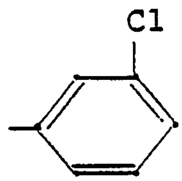 | $i\text{-}C_3H_7$ | 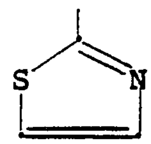 | |
| 6 | 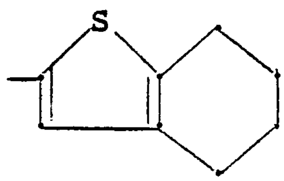 | $CH_3$ | 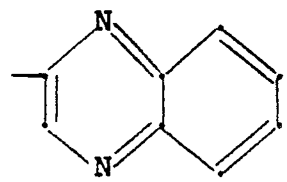 | |
| 7 | 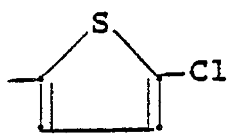 | $CH_3$ | 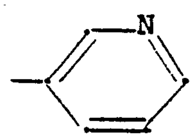 | |
| 8 | 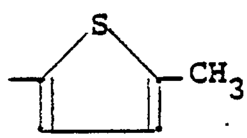 | $CH_3$ | 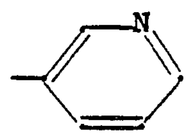 | |
| 9 | 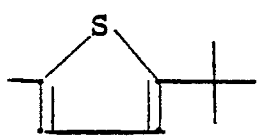 | $CH_3$ | 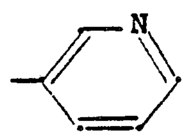 | |
| 10 | 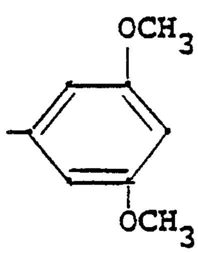 | $CH_3$ | 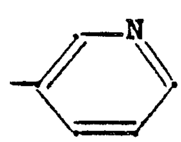 | |

## TABELLE IX

Verbindungen der Formel

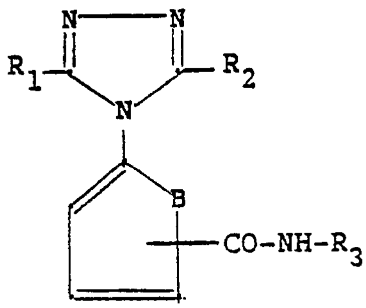

| Nr. | $R_1$ | $R_2$ | $R_3$ | 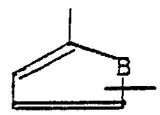 | Fp[°C] |
|---|---|---|---|---|---|
| 1 | 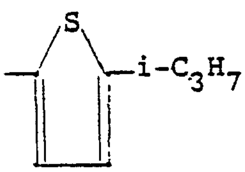 | i-$C_3H_7$ | 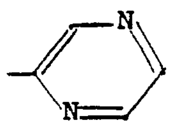 | 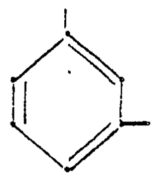 | |
| 2 | 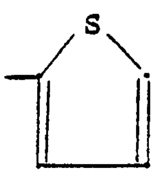 | 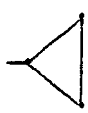 | 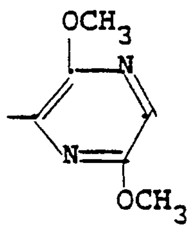 | 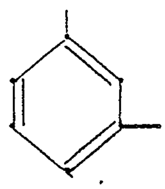 | |
| 3 | 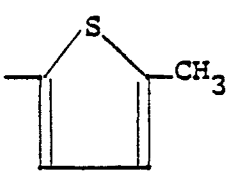 | i-$C_3H_7$ | 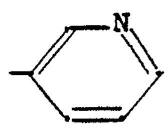 | 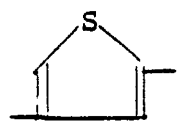 | |

| Nr. | $R_1$ | $R_2$ | $R_3$ | 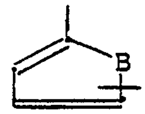 | Fp[°C] |
|---|---|---|---|---|---|
| 4 | 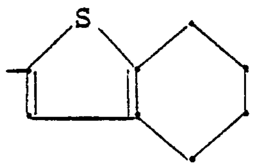 | $CH_3$ | 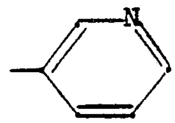 | 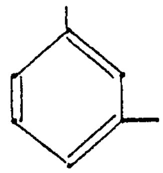 | |
| 5 | 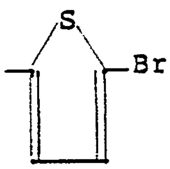 | $CH_3$ | 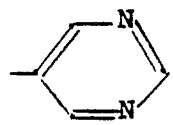 | 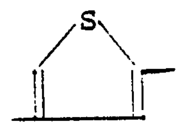 | |
| 6 | 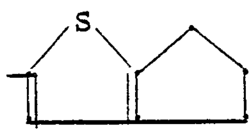 | $CH_3$ | 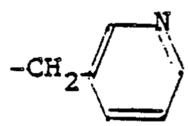 | 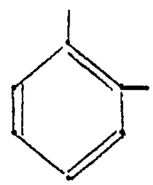 | |

## TABELLE X

Verbindungen der Formel

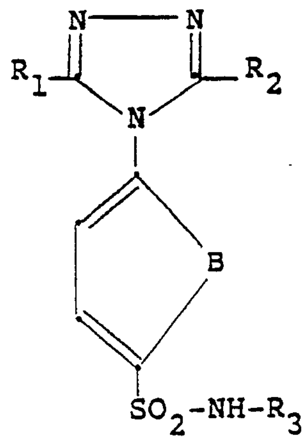

| Nr. | $R_1$ | $R_2$ | $R_3$ | Fp[°C] |
|---|---|---|---|---|
| 1 | 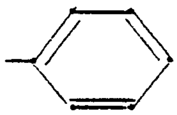 | i-$C_3H_7$ | 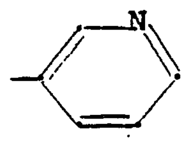 | |
| 2 | 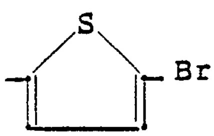 | 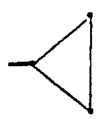 | 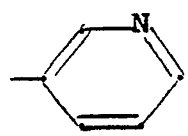 | |
| 3 | 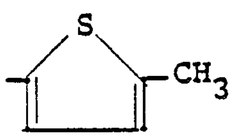 | $CH_3$ | 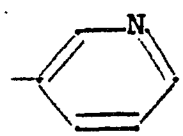 | |
| 4 | 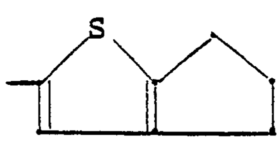 | $CH_3$ | 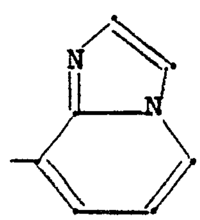 | |

| Nr. | $R_1$ | $R_2$ | $R_3$ | Fp[°C] |
|---|---|---|---|---|
| 5 | 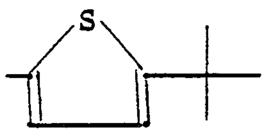 | $CH_3$ | 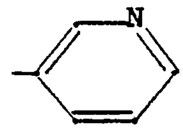 | |
| 6 | 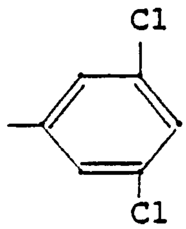 | $CH_3$ | 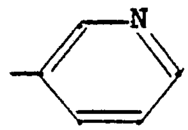 | |
| 7 | 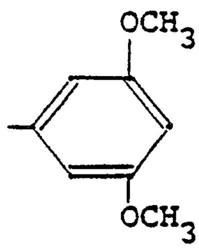 | $CH_3$ | 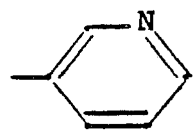 | 195-7 |

## TABELLE XI

Verbindungen der Formel

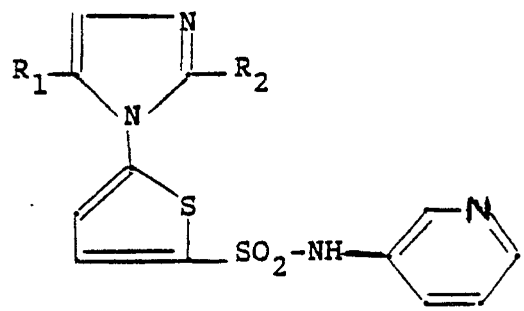

| Nr. | $R_1$ | $R_2$ | Fp[°C] |
|---|---|---|---|
| 1 | 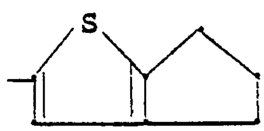 | i-$C_3H_7$ | |
| 2 | 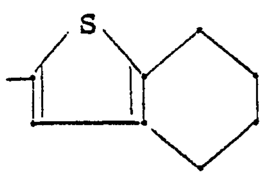 | 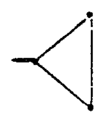 | |
| 3 | 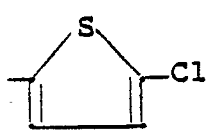 | 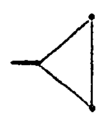 | |
| 4 | 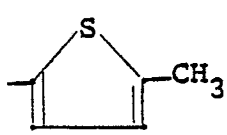 | $CH_3$ | |
| 5 | 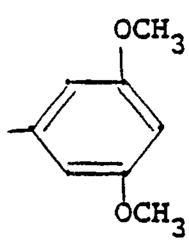 | $CH_3$ | |

TABELLE XII

Verbindungen der Formel

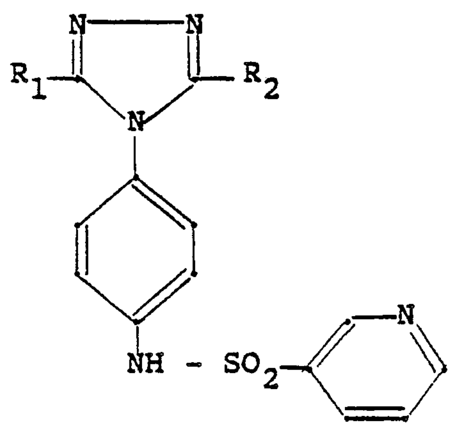

| Nr. | $R_1$ | $R_2$ | Fp[°C] |
|---|---|---|---|
| 1 | 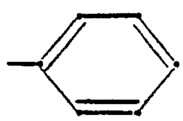 | i-$C_3H_7$ | |
| 2 | 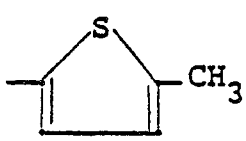 | 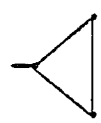 | |
| 3 | 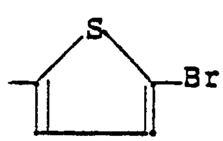 | $CH_3$ | |
| 4 | 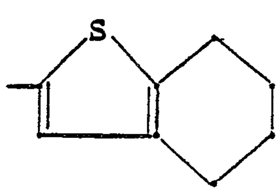 | i-$C_3H_7$ | |

## TABELLE XIII

Verbindungen der Formel

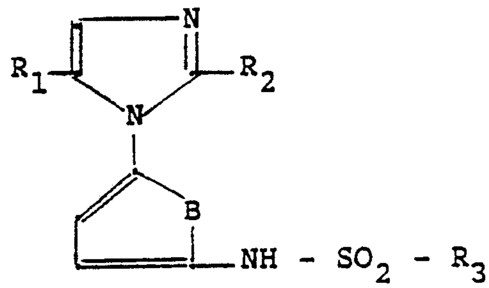

| Nr. | B | $R_1$ | $R_2$ | | Fp[°C] |
| --- | --- | --- | --- | --- | --- |
| 1 | CH=CH | 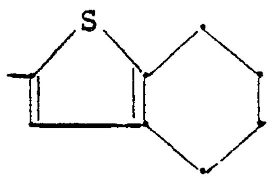 | $i-C_3H_7$ | 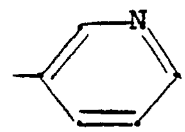 | |
| 2 | S | 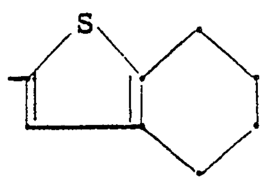 | 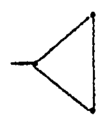 | 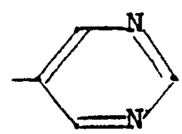 | |
| 3 | CH=CH | 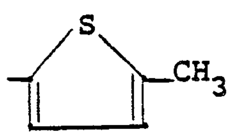 | $CH_3$ | 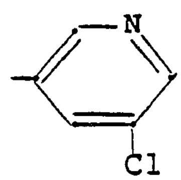 | |
| 4 | CH=CH | 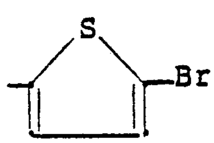 | $n-C_4H_9$ | 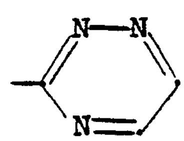 | |
| 5 | S | 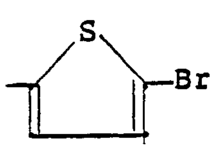 | $C_2H_5$ | 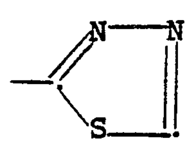 | |

| Nr. | B | $R_1$ | $R_2$ | | Fp[°C] |
|---|---|---|---|---|---|
| 6 | CH=CH | 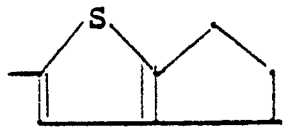 | $CH_3$ | 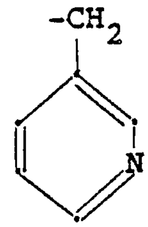 | |
| 7 | S | 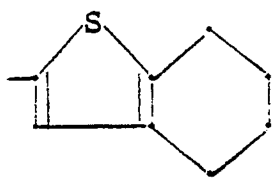 | $i-C_3H_7$ | 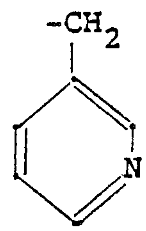 | |

## TABELLE XIV

Verbindungen der Formel

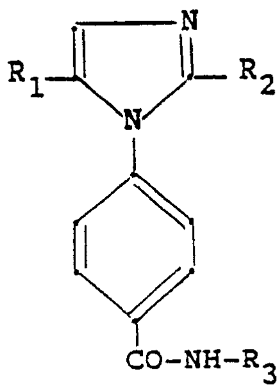

| Nr. | $R_1$ | $R_2$ | $R_3$ | Fp[°C] |
|---|---|---|---|---|
| 1 | 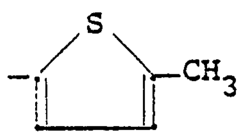 | $CH_3$ | 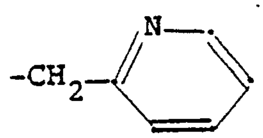 | 178-80 |
| 2 | 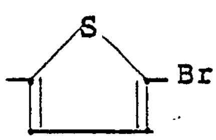 | $CH_3$ | 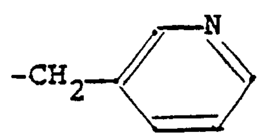 | |
| 3 | 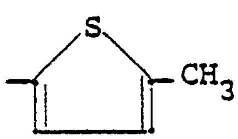 | $i-C_3H_7$ | 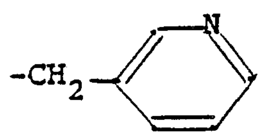 | |
| 4 | 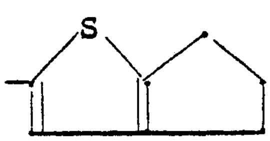 | $i-C_3H_7$ | 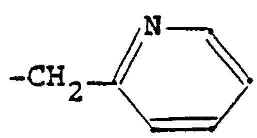 | |

| Nr. | $R_1$ | $R_2$ | $R_3$ | Fp[°C] |
|---|---|---|---|---|
| 5 | 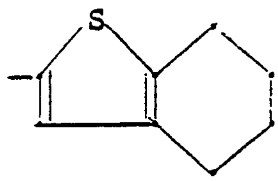 | 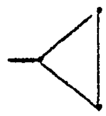 | 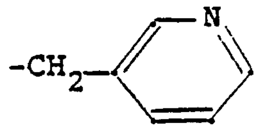 | |
| 6 | 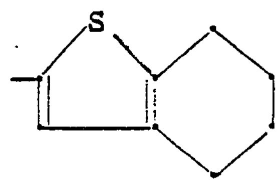 | 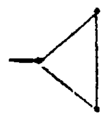 | 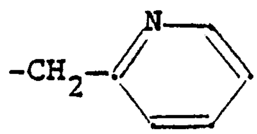 | |

## TABELLE XV

Verbindungen der Formel

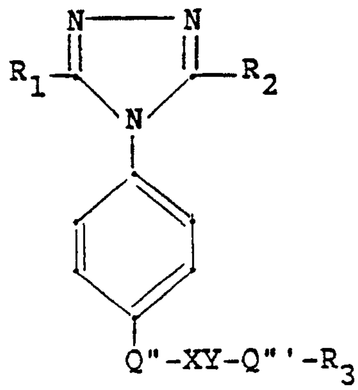

| Nr. | $R_1$ | $R_2$ | Q"-XY-Q"'-$R_3$ | Fp[°C] Base | Salz |
|---|---|---|---|---|---|
| 1 | 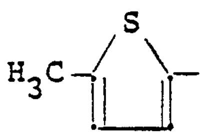 | $CH_3$ | $CONH-CH_2$ 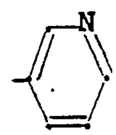 | 100-3 (x 1/2 $H_2O$) | |
| 2 | 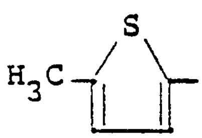 | $CH_3$ | $CONH-(CH_2)_3$ 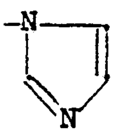 | 110-2 | |
| 3 | 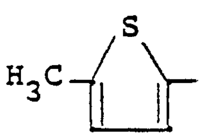 | $CH_3$ | $CONH-CH_2$ 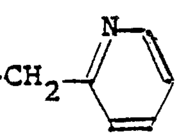 | 178-80 | |
| 4 | 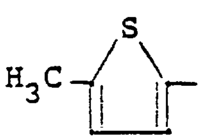 | $CH_3$ | 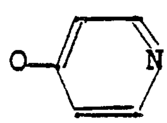 | 146-8 | |

| Nr. | $R_1$ | $R_2$ | Q"-XY-Q"'-$R_3$ | Fp[°C] Base Salz |
|---|---|---|---|---|
| 5 | 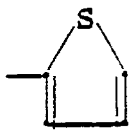 | $CH_3$ | 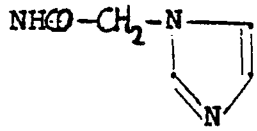 | 190-3 (x $H_2O$) |
| 6 | 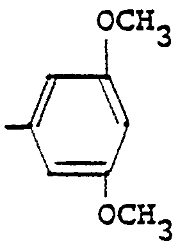 | $CH_3$ | $CON(CH_3)-$ 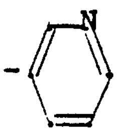 | 166-8 |

## TABELLE XVI

Verbindungen der Formel

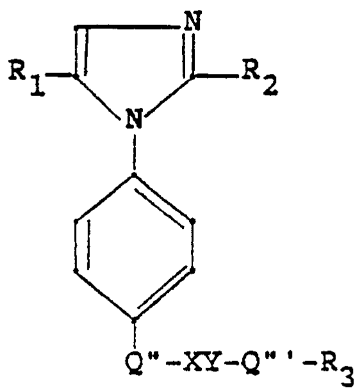

| Nr. | $R_1$ | $R_2$ | Q"-XY-Q"'-$R_3$ | Fp[°C] Base | Salz |
|---|---|---|---|---|---|
| 1 | 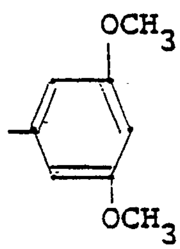 | $CH_3$ | $CONH-CH_2$— 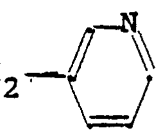 | | |
| 2 | 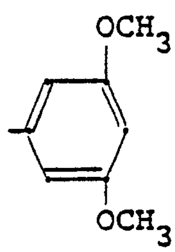 | $OCH_3$ | $CONH-CH_2$— 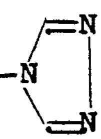 | | |
| 3 | 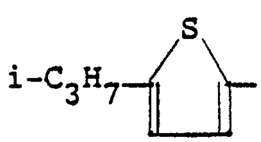 | 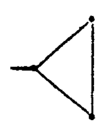 | $CONH-CH_2$— 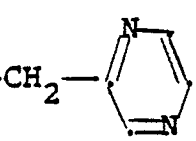 | | |

# TABELLE XVII

Verbindungen der Formel

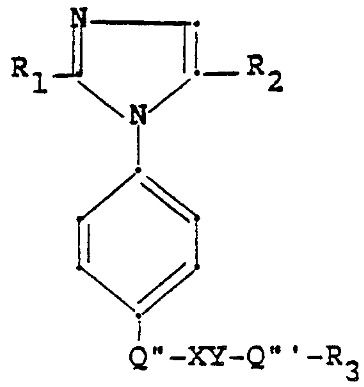

| Nr. | $R_1$ | $R_2$ | Q"-XY-Q"'-$R_3$ | Fp[°C] Base/Salz |
|---|---|---|---|---|
| 1 | 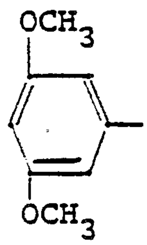 | $C(CH_3)$ | CONH 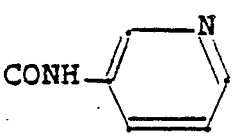 | |
| 2 | Br 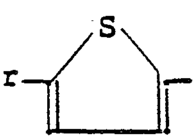 | $OCH_3$ | CONH-$CH_2$)- 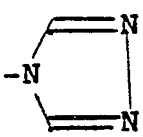 | |
| 3 | 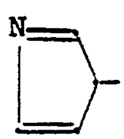 -$CH_2$- | $CH_3$ | $CH_2$CONH 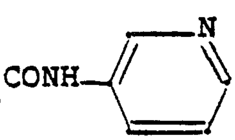 | |
| 4 | 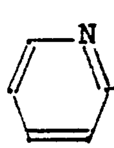 -$CH_2$- | $CH_3$ | NHCONH 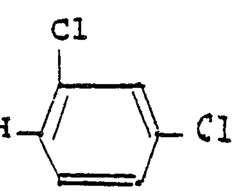 | |

| Nr. | $R_1$ | $R_2$ | $Q''-XY-Q'''-R_3$ | Fp[°C] Base/Salz |
|---|---|---|---|---|
| 5 | 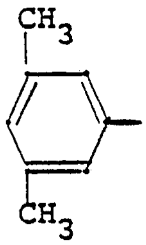 | $CH_2=CH-CH_2$ | 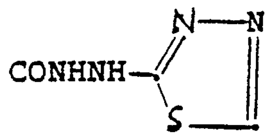 | |

## TABELLE XVIII

| Nr. | Formel | Fp.[°C] Base | Fp.[°C] Salz |
|---|---|---|---|
| 1 | 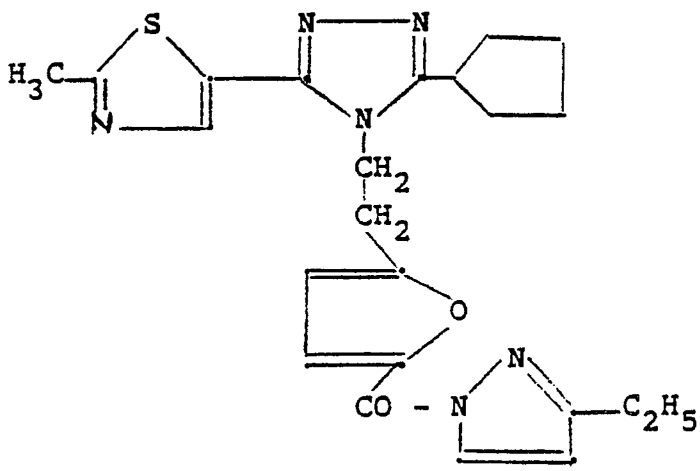 | | |
| 2 | 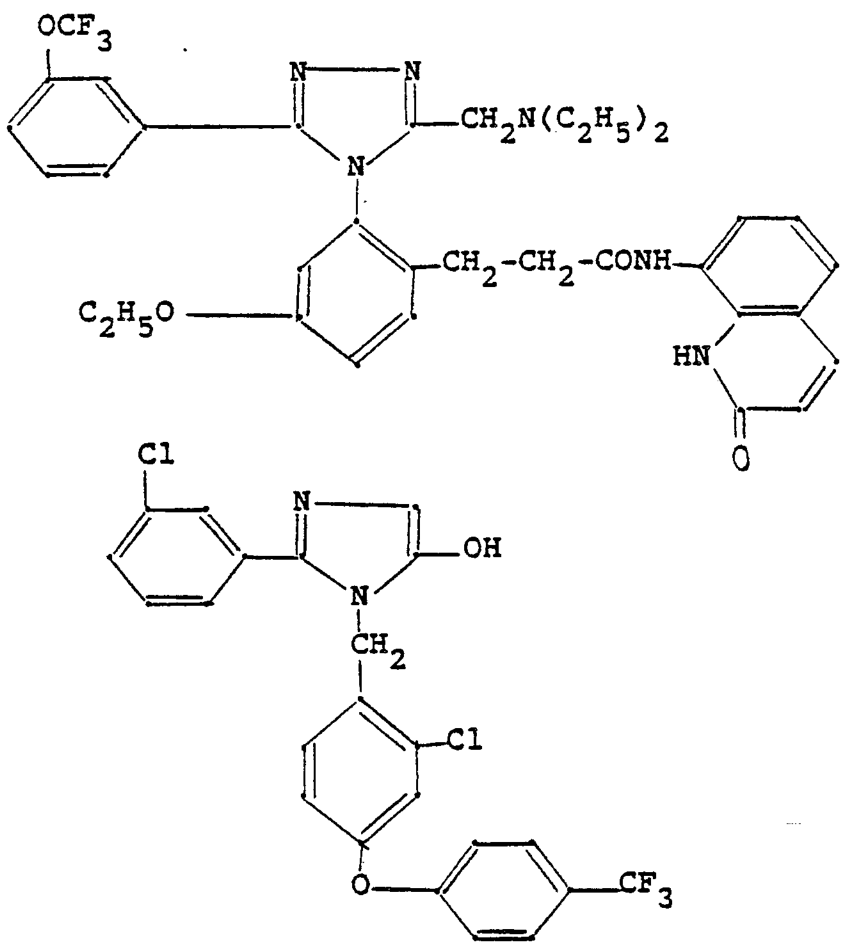 | | |
| 3 | | | |

| Nr. | Formel | Fp.[°C] Base | Fp.[°C] Salz |
|---|---|---|---|
| 4 | 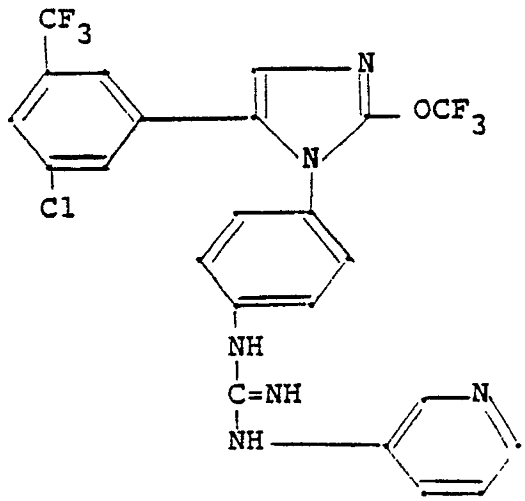 | | |
| 5 | 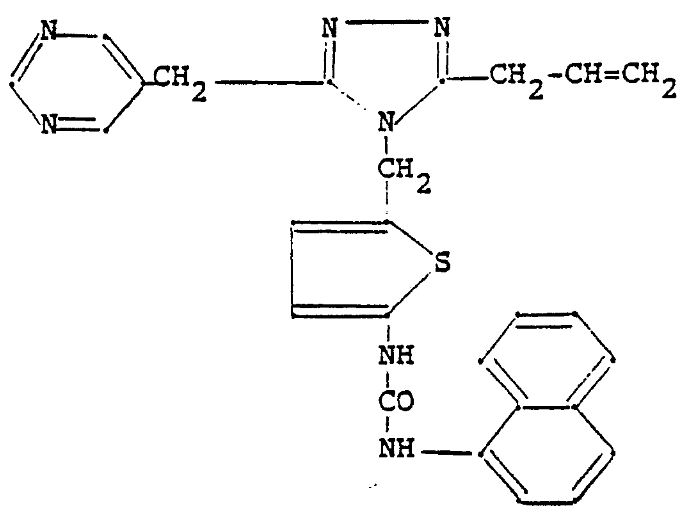 | | |
| 6 | 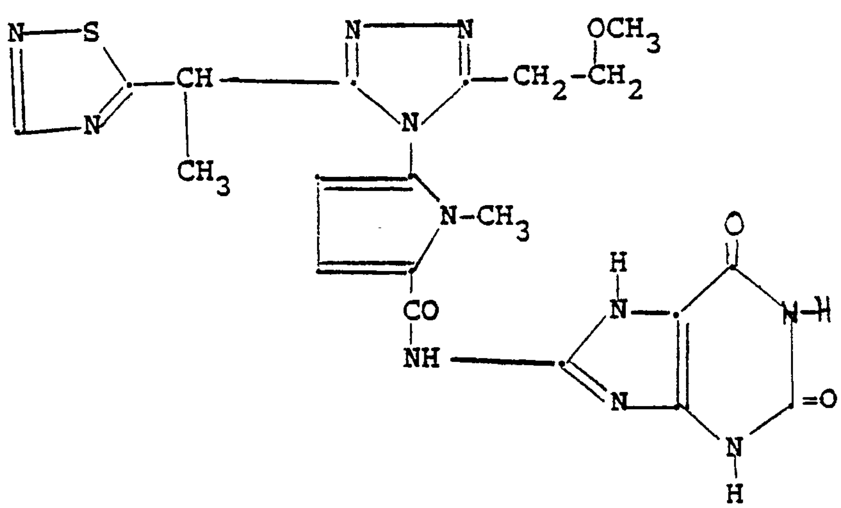 | | |

| Nr. | Formel | Fp.[°C] Base | Salz |
|---|---|---|---|
| 7 | 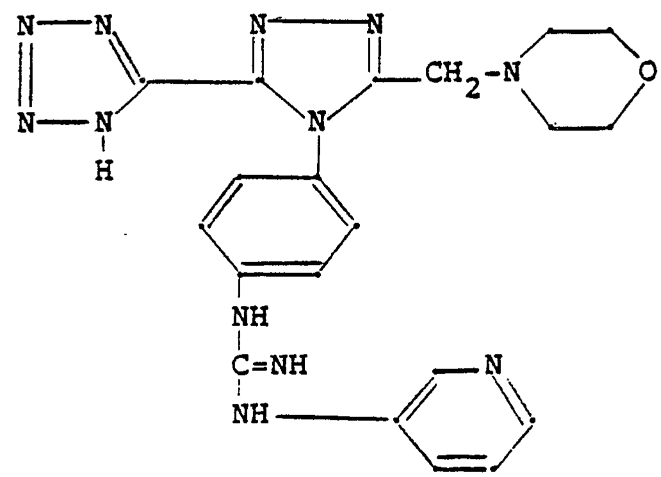 | | |

## Ansprüche

1. Verbindungen der Formel

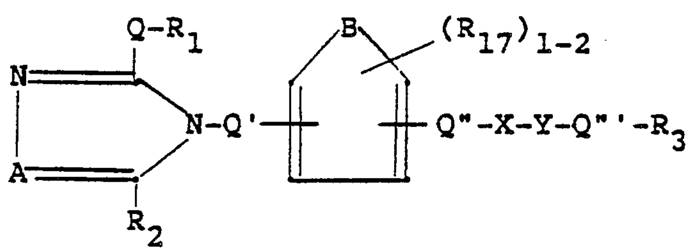

(Ia)

bzw.

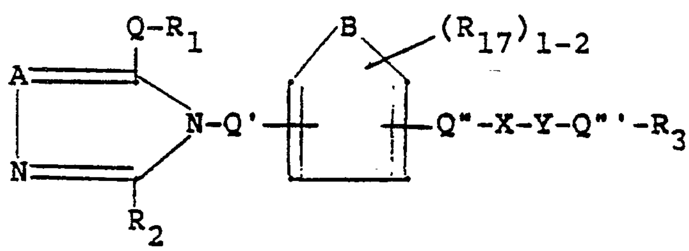

(Ib)

worin

A für N oder CH;

B für einen ein- oder zweigliedrigen Ringbestandteil eines ein- oder mehrkernigen aromatischen oder heteroaromatischen Ringsystems, insbesondere eines solchen, in dem B CH=CH, S, O, $NR_{16}$ bedeutet;

Q, Q', Q", Q‴ für eine Einfachbindung oder $C_1$-$C_3$-Alkylen, Q zusätzlich für O oder $NR_{16}$;

$R_1$ für

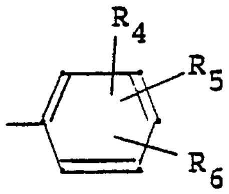 , 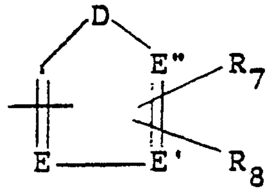 , 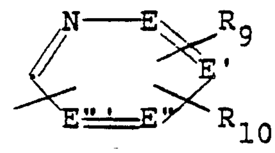

(II) (III) (IV);

stehen

wobei

$R_4$, $R_5$: H, Halogen, $R_{11}$, $OR_{11}$, Aryl, O-Aryl, Aralkyl, O-Aralkyl, $N(R_{11})_2$,

$R_4$ und $R_5$ gemeinsam auch einen gegebenenfalls substituierten ankondensierten $C_5$-$C_7$-Cycloalkenring oder ankondensierten Benzolring oder eine der an benachbarte C-Atome des Benzolrings gebundenen Gruppen -N = CH-NH-, -N = CH-CH = CH-, -O-$CH_2$-$CH_2$-, -O-$CH_2$-$CH_2$-$CH_2$-, -O-$(CH_2)_{1-3}$-O-, -NH-CO-NH-, -N = CH-CH = N-, -HN-CO-$C(R_{16})_2$-O-, -HN-CO-O-, -NH-CO-$CH_2$, -HN-CO-CH = CH--HN-CO-$CH_2$-$CH_2$-;

$R_6$: H, Halogen, $R_{11}$, $OR_{11}$ oder Aryl;

$R_7$: H, Halogen, $R_{11}$, $OR_{11}$, Aryl oder Aralkyl;

$R_8$: H, Halogen, $R_{11}$, $OR_{11}$ Aryl, Aralkyl, $N(R_{11})_2$,

$R_7$ und $R_8$ gemeinsam auch einen ankondensierten gegebenenfalls substituierten $C_5$-$C_7$-Cycloalken-oder Benzolring,

$R_9$,$R_{10}$: H, Halogen, $R_{11}$, $OR_{11}$, $N(R_{11})_2$, Aryl, O-Aryl, Aralkyl, O-Aralkyl,

$R_9$ und $R_{10}$ gemeinsam auch einen gegebenenfalls substituierten ankondensierten $C_5$-$C_7$-Cycloalken-oder Benzolring;

D: S, O, $NR_{16}$;

E,E′,E″,E‴ CH, N, wobei im Fall der Gruppe III nicht mehr als zwei, im Fall der Gruppe IV nicht mehr als drei der Symbole E - E‴ für N stehen;

$R_{11}$: H, $C_1$-$C_{12}$-Alkyl, das sauerstoffunterbrochen oder substituiert sein kann durch bis zu 5 Halogenatome, $N(R_{16})_2$, Aryl, O-Aryl, einen $C_3$-$C_7$-Cycloaliphaten oder einen N-Heterocyclus, der über das Ringstickstoffatom an das Alkyl gebunden ist und der als weiteres Heteroringglied O, S, oder $NR_{16}$ enthalten kann; Alkenyl oder Alkinyl mit insgesamt bis zu 6 C-Atomen

bedeuten;

$R_2$ für H, OH, O-Acyl (mit bis zu 7 C-Atomen), einen gesättigten oder ungesättigten aliphatischen Rest mit bis zu 8 C-Atomen, der O-, S- oder $NR_{16}$-unterbrochen und durch bis zu fünf Halogenatome, OH, O-Acyl, Oxo, Aryl oder O-Aryl substituiert sein kann und der auch über Sauerstoff an den Imidazol- bzw. Triazolring gebunden sein kann; für einen gegebenenfalls durch $R_{16}$, OH, Oxo, $N(R_{16})_2$, substituierten, gesättigten oder ungesättigten, gegebenenfalls über Sauerstoff oder $C_1$-$C_4$-Alkylen an den Imidazol- bzw. Triazolring gebundenen $C_3$-$C_7$-cycloaliphatischen Ring; oder für eine Gruppe $(CH_2)_n$-$NR_aR_b$ (n = 0, 1, 2 oder 3), $R_a$ und $R_b$ H, oder einen gesättigten oder ungesättigten, gegebenenfalls sauerstoffunterbrochenen aliphatischen Rest mit bis zu 6 C-Atomen, der auch OH oder $N(R_{16})_2$ substituiert sein kann; $R_a$ außerdem für einen $C_3$-$C_7$-cycloaliphatischen Rest; die ganze Gruppe $NR_aR_b$ auch für einen 5-7-gliedrigen Ring der als zusätzliches Ringglied O, S oder $NR_{16}$ enthalten kann;

$R_3$ für einen ein- oder mehrkernigen gegebenenfalls substituierten vorzugsweise aromatischen carbocyclischen oder stickstoffhaltigen heterocyclischen Rest steht, wobei letzterer über ein Stickstoff- oder ein Kohlenstoffatom an -Q‴-Y- gebunden sein kann, insbesondere für

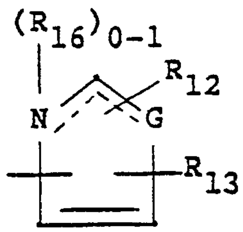

(V)

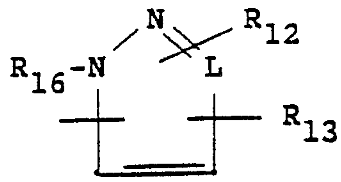

(V')

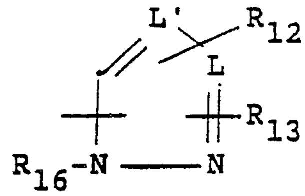

(V")

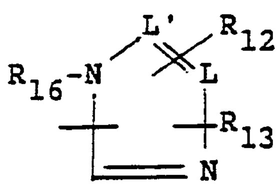

(V"')

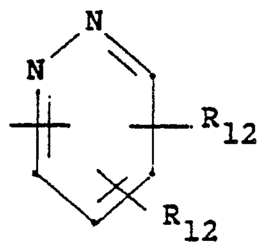

(VI)

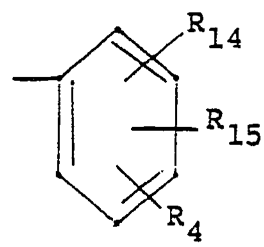

(VII)

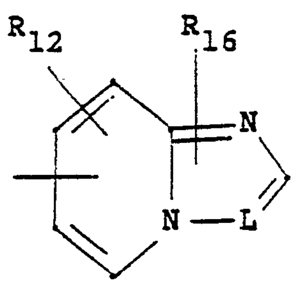

(VIII)

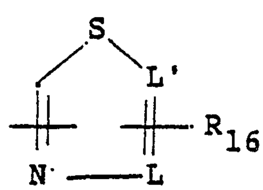

(IX)

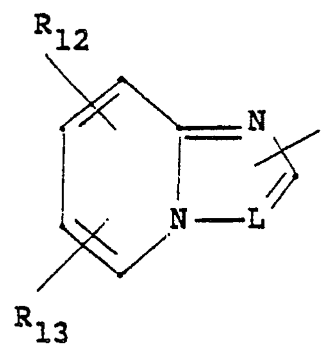

(X)

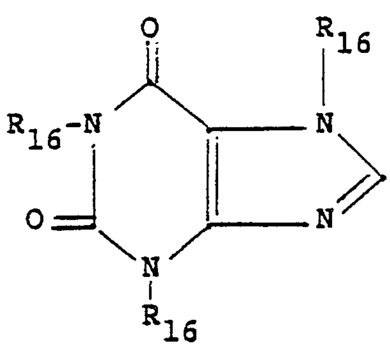

(X')

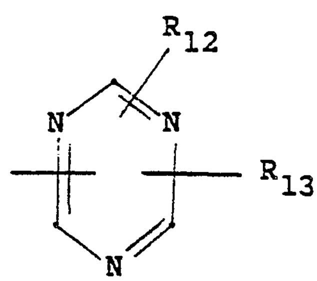

(X")

stehen,

wobei

G: S, O, CH, CH=CH, N=CH, CH=N, N=N, $NR_{16}$;

L,L′: N, $CR_{16}$;

$R_{12}$: H, $(O)_{0-1}$-($C_1$-$C_4$-Alkyl), Aryl, O-Aryl, Aralkyl, Halogen, OH,

$R_{13}$: H, $(O)_{0-1}$-($C_1$-$C_4$-Alkyl), wobei die Alkylketten jeweils OH- oder bis zu fünffach halogensubstituiert sein können, Alkenyl- oder Alkinyl mit bis zu 6 C-Atomen oder Halogen, $R_{12}$ und $R_{13}$ gemeinsam auch einen gegebenenfalls substituierten ankondensierten Benzolring,

$R_{14}$: H, Halogen, CN, OH, $(O)_{0-1}$-($C_1$-$C_4$-Alkyl) (wobei das Alkyl durch bis zu 5 Halogenatome substituiert sein kann), $N(R_{16})_2$, Alkenyl- oder Alkinylreste mit bis zu 6 C-Atomen, CO-($C_1$-$C_4$-Alkyl), CO-Aryl, Aralkyl, Aryl, O-Aryl, einen Rest der Formel III oder IV;

$R_{15}$: H, Halogen, OH, $(O)_{0-1}$-($C_1$-$C_4$-Alkyl), gegebenenfalls durch OH oder bis zu 5 Halogenatome substituiert, $R_{14}$ und $R_{15}$ gemeinsam auch einen gegebenenfalls substituierten ankondensierten homocyclischen oder heterocyclischen Fünf- bis Siebenring, wobei bis zu zwei Ringglieder Heteroatcme aus der Gruppe N, $NR_{16}$, O und S sein können;

$R_{16}$: H, $C_1$-$C_4$-Alkyl

bedeuten;

$R_{17}$ für H, $(0)_{0-1}$-$C_1$-$C_4$-Alkyl, Halogen;

X-Y für eine Einfachbindung, eine zweibindige Brückengruppe der Formel $CONR_{16}$,$CSNR_{16}$,C(NH)-$NR_{16}$,$NR_{16}CO$, $SO_2NR_{16}$ $NR_{16}SO_2$, $CONR_{16}NR_{16}$, $NR_{16}CONR_{16}$, $NR_{16}C(NR_{16})NR_{16}$, $NR_{16}CONR_{16}NR_{16}$, $CO(CH_2)_{1-3}NH$, $NH(CH_2)_{1-3}CO$, $SO_2(CH_2)_{1-3}NH$, $NH(CH_2)_{1-3}SO_2$, und, wenn der Rest $R_3$ über ein Kohlenstoffatom mit Q‴ verknüpft ist und mindestens eine der Gruppen Q″ und Q‴ für eine Einfachbindung steht, auch für CO oder O stehen,

gegebenenfalls in Form einzelner räumlicher Isomerer und ihrer Mischungen und/oder von Säureadditionssalzen und/oder, falls $R_3$ einen Pyridinring umfaßt, in Form von Quartärverbindungen, bei denen die zusätzlichen Gruppen in der Pyridiniumstruktur Niederalkyl oder Aralkyl sind.

2. Verbindungen nach Anspruch 1, worin

B: CH=CH, S, $NR_{16}$ oder O;

$R_1$: eine Gruppe der Formel II, worin

$R_4$, $R_5$, $R_6$ H, $C_1$-$C_4$-Alkyl, $C_1$-$C_4$-Alkoxy oder Halogen bedeuten, $R_4$ außerdem Aryl, Aralkyl, Aryloxy oder $N(R_{16})_2$ sowie $C_5$-$C_{12}$-Alkyl oder -Alkoxy, wenn $R_5$ und $R_6$ H sind, ferner $R_4$ und $R_5$ gemeinsam ankondensiertes $C_5$-$C_7$-Cycloalken, O-$(CH_2)_{1-3}$-O oder N=CH-NH (gebunden an benachbarte C-Atome), und, wenn $R_6$ Wasserstoff ist, auch einen ankondensierten Benzolring;

eine Gruppe der Formel III, worin

D die obige Bedeutung hat, höchstens eines der Symbole E, E′, E″, E‴ N bedeutet, während die übrigen CH sind;

$R_7$, $R_8$ H, $C_1$-$C_4$-Alkyl, $C_1$-$C_4$-Alkoxy, Halogen, ferner, für $R_8$ gleich H, $R_7$ auch $C_5$-$C_{12}$-Alkyl oder Aryl, $R_7$ und $R_8$ gemeinsam auch einen ankondensierten $C_5$-$C_7$ Cycloalken- oder Benzolring bedeuten;

eine Gruppe der Formel IV, worin von den Symbolen E, E′, E″, E‴ nicht mehr als zwei N bedeuten;

$R_9$, $R_{10}$ Halogen, $R_{16}$, $OR_{16}$, $N(R_{16})_2$,

$R_9$ auch Aryl, O-Aryl, Aralkyl und $R_9$ und $R_{10}$ gemeinsam auch einen ankondensierten $C_5$-$C_7$-Cycloalkenring oder Benzolring bedeuten;

$R_2$: H, OH, gesättigter oder ungesättigter aliphatischer Rest mit bis zu 8 C-Atomen, $C_3$-$C_6$-Cycloalkyl, bis zu fünffach halogensubstituiertes $C_1$-$C_3$-Alkyl oder $CH_3OCH_2CH_2$,

$R_3$: eine Gruppe der Formel V, V′, V″, V‴, VII oder IX,

worin

G, L, L′, $R_{16}$ und $R_{17}$ die obige Bedeutung haben;

$R_{11}$: $R_{16}$ ist;

$R_{12}$: $R_{16}$, Aryl oder Halogen,

$R_{13}$: $R_{16}$, Alkenyl mit bis zu 4 C-Atomen,

$R_{12}$ und $R_{13}$ gemeinsam auch einen ankondensierten Benzolring bedeuten;

$R_{14}$: $R_{16}$, Halogen, CN, $CF_3$, CO-($C_1$-$C_4$-Alkyl), $OR_{16}$;

$R_{15}$: $R_{16}$, Halogen oder $OR_{16}$;

$R_4$: die oben als bevorzugt genannte Bedeutung hat;

A, Q, Q′, Q″, Q‴ sowie XY wie oben definiert sind.

3. Verbindungen nach Anspruch 1, worin

A und D die obige Bedeutung haben;

B für CH=CH oder S steht;

Q und Q′ eine Einfachbindung oder $CH_2$ darstellen,

Q″ $C_1$-$C_3$-Alkylen, vorzugsweise jedoch eine Einfachbindung ist;
Q‴ eine Einfachbindung oder, falls XY CO ist, auch $CH_2$;
$R_1$ für die Gruppen II und III steht, wobei
$R_4$, $R_5$, und $R_6$ die oben als bevorzugt genannte Bedeutung haben, ebenso D, E, E′, E″, G, L, L″, $R_3$, $R_{11}$, $R_{14}$, $R_{15}$, $R_{16}$;
$R_7$, $R_8$ H, $C_1$-$C_4$-Alkyl, $C_1$-$C_4$-Alkoxy, $R_8$ auch Aryl, $R_7$ und $R_8$ gemeinsam auch einen ankondensierten Benzolring oder $C_5$-$C_7$-Cycloalkenring darstellen;
$R_2$ für H, $C_1$-$C_3$-Alkyl, Cyclopropyl, Phenyl, $CF_3$ oder Allyl steht;
$R_3$ für eine der Gruppen V, V′, V″, V‴, VII oder IX steht, wobei V bevorzugt

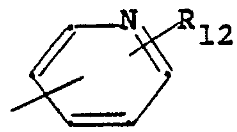

ist;
$R_{12}$ für $R_{16}$, Phenyl, Halogen;
$R_{13}$ für $R_{16}$ oder gemeinsam mit $R_{12}$ auch für einen ankondensierten Benzolring steht;
$R_{17}$ H, $CH_3$, $OCH_3$ oder Cl ist,
XYQ‴ für $CONR_{16}$, $NR_{16}CO$, $SO_2NR_{16}$, $NR_{16}SO_2$, $NR_{16}CONR_{16}$, $CO(CH_2)_{1-3}NH$, $COCH_2$ oder O steht.

4. Verbindungen nach Anspruch 1 mit der Formel

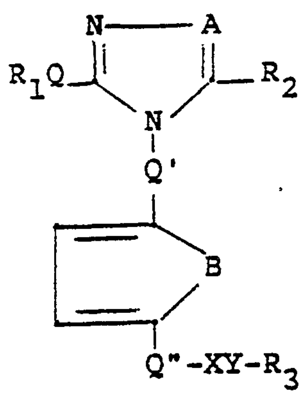

und

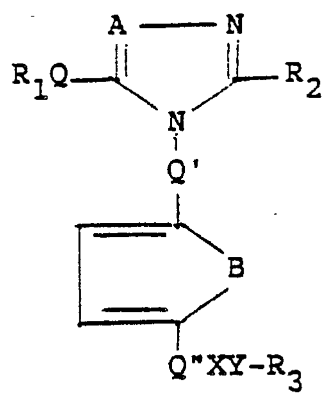

(I'a) (I'b)

A: CH,N;
B: CH=CH, S;
Q, Q′, Q″: Einfachbindung, $CH_2$;
$R_1$:

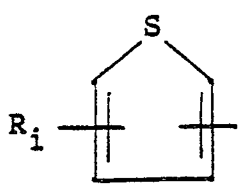

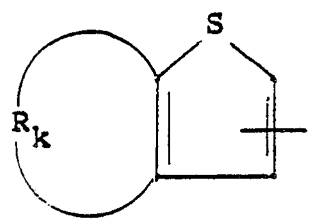

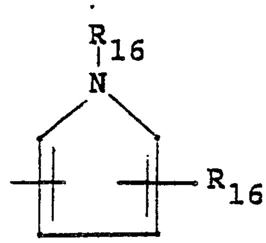

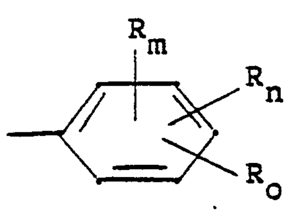

$R_i$: $C_1$-$C_4$-Alkyl, Cl, Br, $C_3$-$C_7$-Cycloalkyl;
$R_k$: ankondensierter $C_5$-$C_6$-Cycloalken- oder Benzolring;
$R_m$, $R_n$, $R_o$: H, Cl, $CH_3$, $C_{1-4}$-Alkoxy, $CF_3$, $N(R_{16})_2$, für den Fall $R_o$ gleich H sind $R_m$ und $R_n$ gemeinsam außerdem ein ankondensierter Benzolring oder $OCH_2O$, gebunden an benachbarte C-Atome;
$R_{16}$: H, $C_1$-$C_4$-Alkyl, $N(R_{16})_2$ vor allem $N(CH_3)_2$ und $N(C_2H_5)_2$;
$R_2$: $CH_3$, $C_2H_5$;
XY: CONH, NHCO, $SO_2NH$, NHCONH, $COCH_2$, $CH_2O$;
$R_3$:

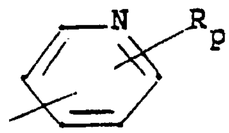
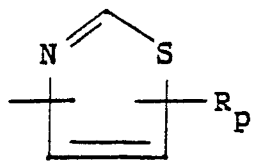
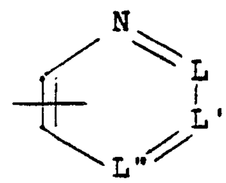

wobei eines der Symbole L, L′, L″ für N, die anderen für CH stehen und $R_p$ H oder $CH_3$ ist.

5. Verbindungen nach Anspruch 1 mit der Formel

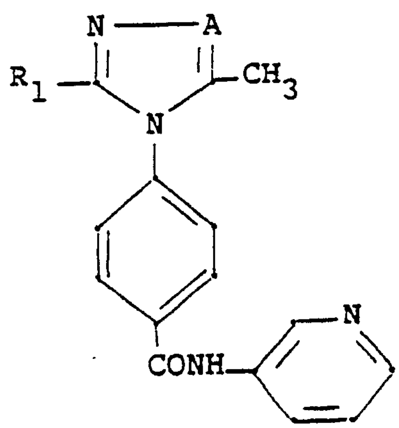

(I"a)

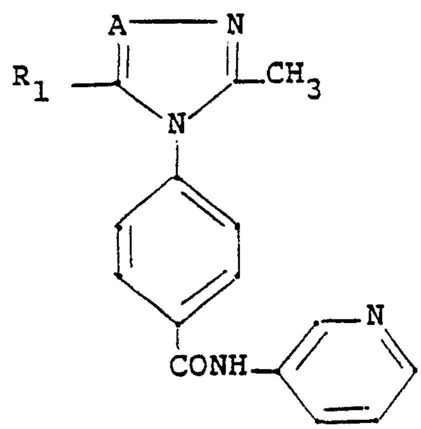

(I"b)

worin
$R_1$: Naphthyl sowie

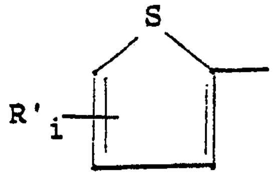
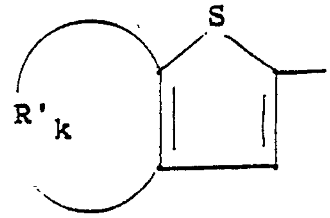
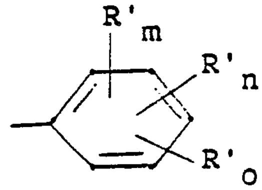

A: N oder CH
$R'_i$: $C_1$-$C_4$-Alkyl, Cl, Br,
$R'_k$: ankondensierter Cyclopenten- oder Cyclohexenring;
$R'_m$: H, Cl, $CH_3O$, Cl, $CF_3$, $CH_3$;
$R'_n$: H, $CH_3O$, Cl, $CH_3$; beide zusammen $OCH_2O$ an benachbarten C-Atomen;
$R'_o$: H, $CH_3O$, $CH_3$.

6. Verbindungen nach Anspruch 1, mit der Formel

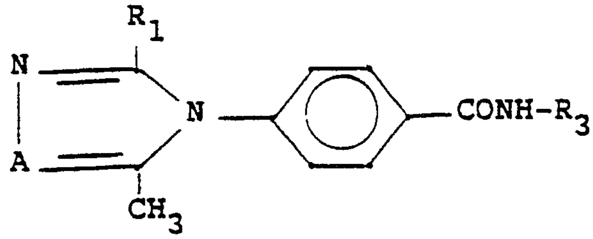

bzw.

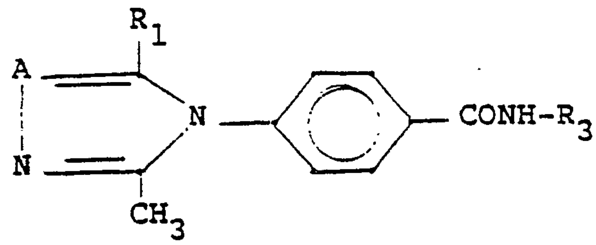

worin

A CH oder N,

$R_1$

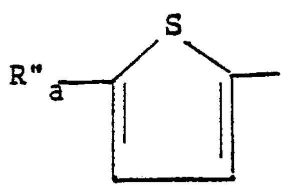 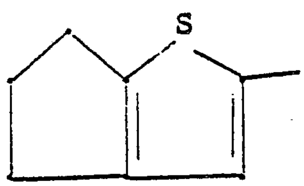 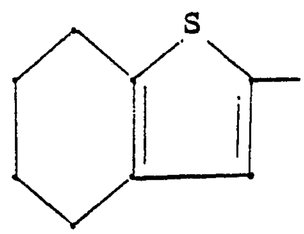

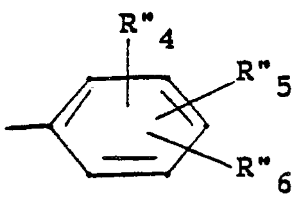

$R_3$

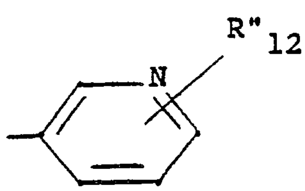 , 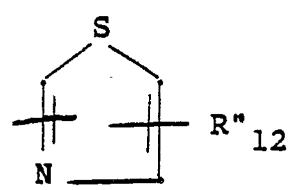

bedeuten, wobei

$R''_a$ $C_1$-$C_4$-Alkyl, Cl oder Br;

$R''_4$ H, $CH_3$, $OCH_3$, Cl, $CF_3$; $R''_5$ H, $CH_3$, $OCH_3$, Cl;

$R''_5$ H, $OCH_3$;

$R''_{12}$ H, $CH_3$, Cl, im Fall des Thiazols auch einen ankondensierten Benzolring darstellt.

7. Als Stoffe mit PAF-antagonistischer Wirkung die Verbindungen nach den Ansprüchen 1 bis 5.

8. Pharmazeutische Zubereitungen, gekennzeichnet durch einen Gehalt an einer Verbindung nach einem der Ansprüche 1 bis 7.

9. Verwendung von Verbindungen nach den Ansprüchen 1 bis 7 bei der Behandlung von Krankheiten, an denen PAF beteiligt ist, insbesondere entzündlichen und allergischen Vorgängen sowie Autoimmunkrankheiten.

10. Verfahren zur Herstellung der Verbindungen nach Anspruch 1 bis 7 nach an sich bekannten Verfahren, dadurch gekennzeichnet, daß man
a) Acylamidrazone der Formel

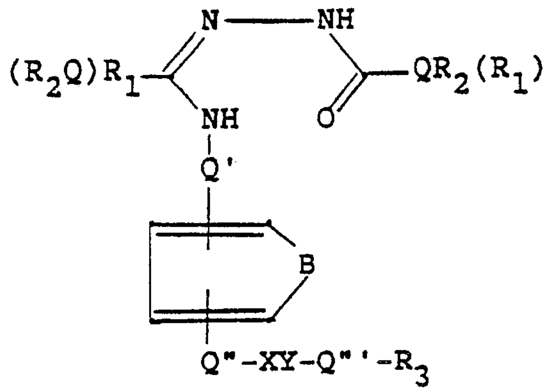

(XI),

in der $R_1$, $R_2$, $R_3$, B, Q, Q′, Q″, Q‴, X und Y die obige Bedeutung haben, ohne Lösungsmittel oder in einem inerten Lösungsmittel erhitzt,
oder daß man
b) N-Chloramidine der Formel XXI mit Enaminen bzw. Silylenolethern der Formel XXII umsetzt

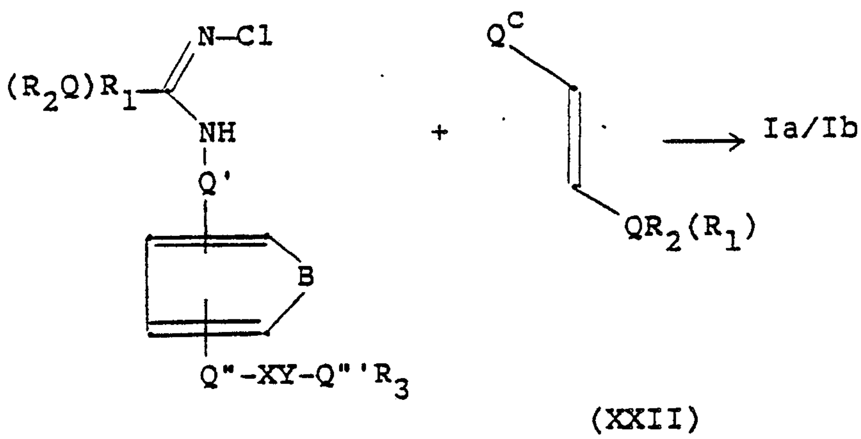


(XXII)

(XXI)

$Q^c$ = N(Niederalkyl)$_2$, OSi(Niederalkyl)$_3$
oder daß man
c) substituierte Amide XXIII mit Aminen der Formel XXIV in Gegenwart von $PCl_3$ in der Wärme umsetzt:

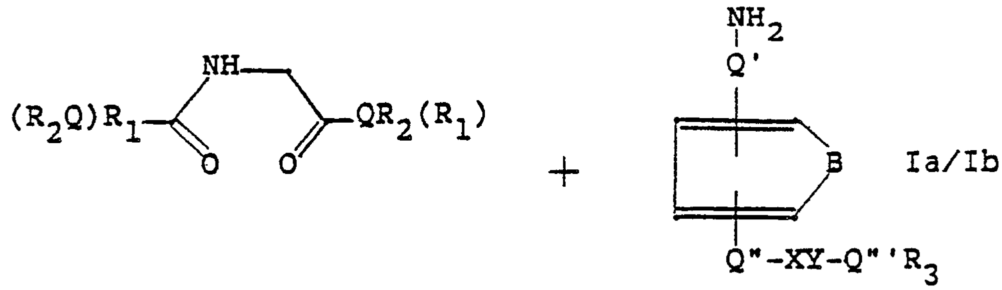

(XXIII) (XXIV)

oder daß man

d) Verbindungen XXV mit Aminen XXVI umsetzt:

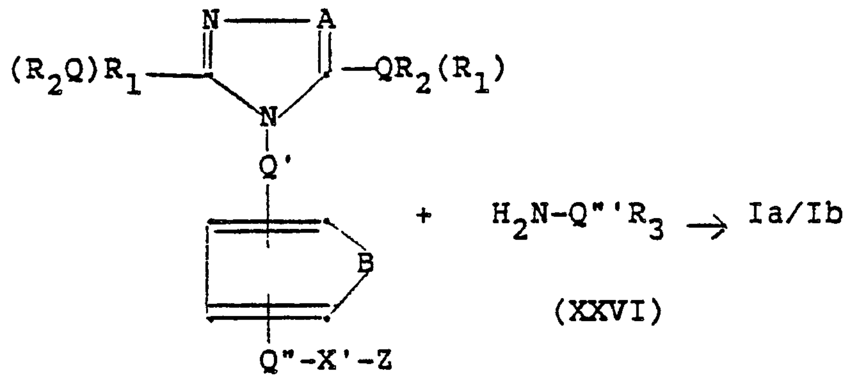

(XXVI)

(XXV)

oder daß man

e) Amine XXVII mit Carbonsäuren bzw. Carbonsäurederivaten XXVIII bzw. mit Isocyanaten XXIX nach üblichen Methoden entsprechend Verfahren 4 umsetzt:

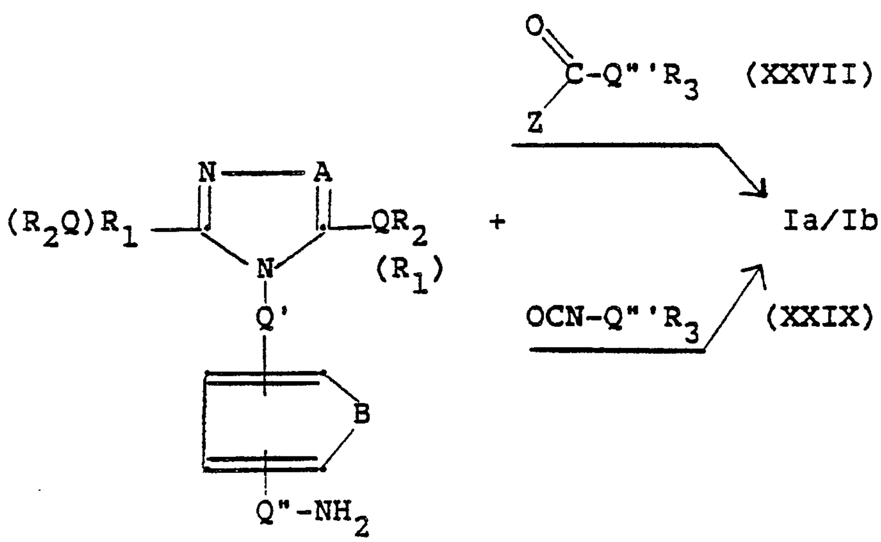

(XXVII) (XXIX)

(XXVII)

(A, B, Q, Q′, Q″, Q‴, $R_1$, $R_2$, $R_3$ und Z haben dabei die oben angegebene Bedeutung),
und daß man gewünschtenfalls nach Verfahren a) bis e) zunächst erhaltene Basen in üblicher Weise in Säureadditionssalze überführt, zunächst erhaltene Salze in freie Basen und/oder daß man gegebenenfalls in

solchen Verbindungen, in denen $R_3$ einen Pyridinring umfaßt, in üblicher Weise am Pyridinstickstoff mit Niederalkyl oder Aralkyl quarternisierte Verbindungen herstellt.

Europäisches Patentamt

**EUROPÄISCHER TEILRECHERCHENBERICHT,** der nach Regel 45 des Europäischen Patent-übereinkommens für das weitere Verfahren als europäischer Recherchenbericht gilt

Nummer der Anmeldung

EP 89 10 5570

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | FR-A-2 183 807 (UPJOHN)<br>* Insgesamt *<br>-- | 1-8<br>10 | C 07 D 233/54<br>C 07 D 249/08<br>A 61 K 31/41<br>C 07 D 409/14<br>C 07 D 401/12<br>C 07 D 403/12<br>C 07 D 401/14 |
| X | US-A-3 842 089 (J.B. HESTER)<br>* Insgesamt *<br>-- | 1-8,<br>10 | |
| Y | FR-A-2 539 127 (ROUSSEL-UCLAF)<br>* Insgesamt *<br>-- | 1-8,<br>10 | |
| Y | FR-A-2 269 938 (DELANESE)<br>* Insgesamt *<br>--<br>./. | 1-8,<br>10 | |

RECHERCHIERTE SACHGEBIETE (Int. Cl.4)

C 07 D 233/00
C 07 D 249/00
C 07 D 401/00
C 07 D 403/00
C 07 D 409/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 5,6
Unvollständig recherchierte Patentansprüche: 1-4,7,8,10
Nicht recherchierte Patentansprüche: 9
Grund für die Beschränkung der Recherche:

Patentansprüche 1-4,7,8,10: Durch die Vielfalt und Verschiedenheit der Substituenten sowie deren gegenseitigen Verknüpfungen in den Formeln Ia und Ib, wird der Umfang des beanspruchten Schutzrechtes nicht klar definiert. Die weiter unten genannten Ansprüche entsprechen darum nicht den Anforderungen des Artikels 84 des Europäischen Patentübereinkommens:

./.

| Recherchenort | Abschlußdatum der Recherche | Prüfer |
|---|---|---|
| Den Haag | 20-06-1989 | ALLARD |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03.82

Europäisches Patentamt

**EUROPÄISCHER TEILRECHERCHENBERICHT,**
der nach Regel 45 des Europäischen Patentübereinkommens für das weitere Verfahren als europäischer Recherchenbericht gilt

Nummer der Anmeldung

EP 89 10 5570

-2-

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0 255 028 (BOEHRINGER INGELHEIM)<br>* Insgesamt * | 1-8, 10 | |
| | -- | | |
| Y | EP-A-0 254 245 (BOEHRINGER INGELHEIM)<br>* Insgesamt * | 1-8, 10 | |
| | ------- | | |

RECHERCHIERTE SACHGEBIETE (Int. Cl.4)

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.
Vollständig recherchierte Patentansprüche:
Unvollständig recherchierte Patentansprüche:
Nicht recherchierte Patentansprüche:
Grund für die Beschränkung der Recherche:

Patentanspruch 9:
Verfahren zur chirurgischen oder therapeutischen Behandlung des menschlichen oder tierischen Körpers (siehe Art. 52(4) des Europäischen Patentübereinkommens).

| Recherchenort | Abschlußdatum der Recherche | Prüfer |
|---|---|---|
| | | |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument